# EUROPEAN PATENT APPLICATION

(11) **EP 1 270 724 A2**
(43) Date of publication of application: **02.01.2003**
(21) Application number: 02013517.4
(22) Date of filing: 18.06.2002
(51) Int. Cl.: C12N 15/12, C12N 15/11, C07K 14/705, C07K 16/28, A61K 38/17, C12Q 1/68

(54) **Guanosine triphosphate-binding protein coupled receptors**

(30) Priority: 18.06.2001 JP 2001246789
(71) Applicant: National Institute of Advanced Industrial Science and Technology, Tokyo 100-8901 (JP); Center for Advanced Science and Technology Incubation, Ltd., Tokyo 100-0005 (JP)
(72) Inventor: Suwa, Makiko, AIST Tokyo Waterfront, 2-41-6, Aomi, Koto-ku, Tokyo 135-0064 (JP); Asai, Kiyoshi, AIST Tokyo Waterfront, 2-41-6, Aomi, Koto-ku, Tokyo 135-0064 (JP); Akiyama, Yutaka, AIST Tokyo Waterfront, 2-41-6, Aomi, Koto-ku, Tokyo 135-0064 (JP); Aburatani, Hiroyuki, Musashino-shi, Tokyo 180-0003 (JP)
(74) Representative: VOSSIUS & PARTNER

(57) **Abstract**

The object of the present invention is to provide a technique for efficiently extracting GPCR sequences from human genome sequences, thereby comprehensively identifying novel GPCRs. An original automatic system for identifying GPCR sequences is disclosed, and 1035 novel GPCRs are successfully identified from the entire human genome by utilizing the system.

## Description

### FIELD OF THE INVENTION

The present invention relates to novel polypeptides belonging to the guanosine triphosphate-binding protein coupled receptor (hereinafter, abbreviated as "GPCR") family, polynucleotides encoding said polypeptides, as well as production and use of the same.

### BACKGROUND OF THE INVENTION

More than 90% of drugs developed by drug industries in the world so far, have targeted interactions in the extracellular spaces, and a majority of these drugs target the GPCRs that comprise seven transmembrane helices (Baldwin J. M., Curr. Opin. Cell Biol. 6: 180-190 (1994); Strader C. D. et al., FASEB. J. 9: 745-754 (1995); Bockaert J., Pin J. P., EMBO. J. 18: 1723-1729 (1999)). Therefore, GPCRs are one of the most important targets in finding genes for designing drugs. The GPCRs are involved in the signal transduction induced by specific ligands, such as adrenaline and acetylcholine, and characteristics of the binding mechanisms thereof have been actively investigated by conducting experiments (Watson S. & Arkinstrall S., The G-protein Linked receptor Facts Book (Academic Press, London)).

However, despite the vast data sources, such as cDNAs, ESTs, and microarray analyses, that have been obtained, only a limited number of novel sequences of the family have been discovered (Lee D. K. et al., Brain Res. Mol. Brain Res. 31: 13-22 (2001) ; Mizushima K. et al., Genomics. 69: 314-321 (2000) ; Matsumoto M. et al., Gene. 28: 183-189 (2000) ; Marchese A. et al., Trends Pharmacol. Sci. 20: 447 (1999); Lee D. K., FEBS. Lett. 446: 103-107 (1999); Yonger R. M. et al., Genome Research. 11: 519-530 (2001); Horn F. et al., Nucleic Acids Res. 29: 346-349 (2001)). Even the large-scale classification of known GPCR sequences, such as GPCRdb (Lee D. K. et al., Brain Res. Mol. Brain Res. 31: 13-22 (2001)) and collections by PSI-BLAST (Josefson L. G., Gene. 239: 333-340 (1999)), have not led to a broadscale elucidation at the level of the entire genome.

Therefore, it is important to elucidate the GPCR families as a whole by scanning human genomic sequences, wherein more than 90% of all the sequences thereof have been already determined (International Human Genome Sequencing Consortium. Initial sequencing and analysis of the human genome. Nature 409; 860-921 (2001); Venter J. C. et al., Science 291: 1304-1351 (2001)).

### SUMMARY OF THE INVENTION

This need in the art led to the present invention, and the object of the present invention is to develop an automated technique for efficiently extracting GPCR sequences from the human genome sequences and thereby inclusively identifying novel GPCRs.

Another object of the present invention is to provide a use for the newly identified GPCRs. As one preferred embodiment of the use of the novel GPCRs, this invention provides for the use of GPCRs to screen drug candidate compounds such as ligands, etc. Moreover, as another preferred embodiment for the use of the novel GPCRs, this invention provides a method for testing disorders based on mutations and expression aberrations of the novel GPCRs as an indicator.

Furthermore, this invention provides a use for the novel GPCRs or molecules that control the activities thereof, in the treatment of disorders.

To accomplish the objects described above, first, the present inventors carefully evaluated analytical methods for sequence search (Altschul S. F. et al., Nucleic Acids Res. 25: 3389-3402 (1997)), motif and domain attribution (Bateman A. et al., Nucleic Acids Res. 28: 263-266 (2000) ; Bairoch A., Nucleic Acids Res. 20: 2013-2018 (1992)), and transmembrane helix prediction (Hirokawa T. et al., Bioinformatics, 14: 378-379 (1998)), and then, developed an automated system for identifying GPCR sequences from the whole human genome. This automated system comprises the following three steps

The first step is to predict genes. More specifically, translation of the genomic sequences into amino acid sequences. The prediction of a gene can be achieved to a certain extent by resorting to 6-frame development of nucleotide sequences, since most of the known GPCR genes contain no introns. On the other hand, for sequences with multiple exons, it is necessary to predict the entire gene structure using a gene-finding program.

The second step consists of a threefold analysis of the amino acid sequences. More specifically, this step comprises: (1) searching for corresponding sequences in known GPCR databases; (2) attributing the motif and domain; and (3) predicting the transmembrane helix (TMH). The former two procedures are used to find closely related GPCR homologues, while the TMH prediction is used to find remote GPCR homologues. Subsequently, candidate sequences are screened by taking the analysis results of the three analyses as a logical sum. In order to maximize the number of candidate sequences at this screening step, the present inventors have used the logical sum of the results of the analyses.

The third step is to further refine the quality of the candidate genes by eliminating overlapping sequences from the second step, and merging fragmented sequences separated by misprediction.

According to this automated system, GPCR sequences can be efficiently and inclusively identified. A further great advantage of the automated system is that it can identify even GPCR sequences consisting of multiple exons and remote homologous sequences, which have been difficult to find by conventional methods.

Using the automated system of the present invention, the inventors have successfully identified 1035 novel GPCR sequences from the whole human genome, such sequences guaranteed with a high confidence to be members of the GPCR family. The discovery of such novel GPCR sequences enables the screening of ligands, antagonists and agonists, which are expected to be useful as drugs. Additionally, GPCRs are thought to have important functions *in vivo.* Thus, aberrations in the expression and function thereof may be the cause of a variety of disorders. Therefore, it is possible to analyze and evaluate such disorders using as an indicator inappropriate functions or expressions of the identified GPCRs. The identified GPCRs, polynucleotides encoding them, and ligands, antagonists, or agonists of the identified GPCRs may function as preferred therapeutic agents for such disorders.

Accordingly, the present invention relates to novel GPCRs and genes encoding them, as well as methods for producing and using same. More specifically, the present invention provides the following:
(1) a polynucleotide encoding a guanosine triphosphate-binding protein coupled receptor selected from the group of:
   (a) a polynucleotide encoding a polypeptide comprising an amino acid sequence of any even-numbered SEQ ID NOs from SEQ ID NO: 2 to SEQ ID NO: 2070;
   (b) a polynucleotide comprising a coding region of the nucleotide sequence of any odd-numbered SEQ ID NOs from SEQ ID NO: 1 to SEQ ID NO: 2069;
   (c) a polynucleotide encoding a polypeptide comprising an amino acid sequence of any even-numbered SEQ ID NOs from SEQ ID NO: 2 to SEQ ID NO: 2070 wherein one or more amino acid residues are substituted, deleted, added and/or inserted; and
   (d) a polynucleotide hybridizing under stringent conditions with a DNA consisting of a nucleotide sequence of any odd-numbered SEQ ID NOs from SEQ ID NO: 1 to SEQ ID NO: 2069;
(2) a polynucleotide encoding a fragment of a polypeptide comprising the amino acid sequence of any even-numbered SEQ ID NOs from SEQ ID NO: 2 to SEQ ID NO: 2070;
(3) a vector comprising the polynucleotide of (1) or (2);
(4) a host cell retaining the polynucleotide of (1) or (2), or the vector of (3);
(5) a polypeptide encoded by the polynucleotide of (1) or (2);
(6) a method for producing the polypeptide of (5) , comprising the step of culturing the host cell of (4), and recovering the produced polypeptide from said host cell or culture supernatant thereof;
(7) an antibody binding to the polypeptide of (5) ;
(8) a method of identifying a ligand of the polypeptide of (5), comprising the steps of:
   (a) contacting a candidate compound with the polypeptide of (5), cell expressing the polypeptide of (5), or cytoplasmic membrane of the cell; and
   (b) detecting whether the candidate compound binds to the polypeptide of (5), cell expressing the polypeptide of (5), or cytoplasmic membrane thereof;
(9) a method for identifying an agonist of the polypeptide of (5), comprising the steps of:
   (a) contacting a candidate compound with the cell expressing the polypeptide of (5); and
   (b) detecting whether the candidate compound induces a signal that indicates the activation of the polypeptide of (5);
(10) a method for identifying an antagonist of the polypeptide of (5), comprising the steps of:
   (a) contacting a cell expressing the polypeptide of (5) with an agonist of the polypeptide of (5) in the presence of a candidate compound; and
   (b) detecting whether the intensity of the signal that indicates the activation of the polypeptide of (5) is reduced or not by comparing with the signal detected in the absence of the candidate compound;
(11) a ligand identified by the method of (8);
(12) an agonist identified by the method of (9);
(13) an antagonist identified by the method of (10);
(14) a kit for use with the method of any one of (8) to (10), comprising at least one molecule selected from the group:
   (a) the polypeptide of (5); and
   (b) the host cell of (4) or cytoplasmic membrane thereof;
(15) a pharmaceutical composition for treating a patient, who is in need of increased activity or expression of the polypeptide of (5), comprising an effective amount of the molecule for the treatment selected from the group of:
   (a) an agonist of the polypeptide of (5) ;
   (b) the polynucleotide of (1) or (2); and
   (c) the vector of (3) ;
(16) a pharmaceutical composition for treating a patient, whose activity or expression of the polypeptide of (5) needs to be suppressed, comprising an effective amount of the molecule for the treatment selected from the group of:
   (a) an antagonist of the polypeptide of (5); and
   (b) a polynucleotide suppressing the expression of a gene encoding the endogenous polypeptide of (5) *in vivo;*
(17) a method for testing a disorder associated with the aberration in the expression of a gene encoding the polypeptide of (5) or the aberration in the activity of the polypeptide of (5), comprising the step of detecting a mutation in the gene or in the expression control region thereof in the subject;
(18) the method of (17), comprising the steps of:
   (a) preparing a DNA sample from a subject;
   (b) isolating from the sample the DNA encoding the polypeptide of (5) or the expression control region thereof;
   (c) determining the nucleotide sequence of the isolated DNA; and
   (d) comparing the nucleotide sequence of DNA determined in step (c) with that of a control;
(19) the method of (17), comprising the steps of:
   (a) preparing a DNA sample from a subject;
   (b) cleaving the prepared DNA sample with a restriction enzyme ;
   (c) separating DNA fragments according to the sizes thereof; and
   (d) comparing the detected sizes of the DNA fragments with those of a control;
(20) the method of (17), comprising the steps of:
   (a) preparing a DNA sample from a subject;
   (b) amplifying in the sample the DNA encoding the polypeptide of (5) or the expression control region thereof;
   (c) cleaving the amplified DNAs with a restriction enzyme;
   (d) separating the DNA fragments according to the sizes thereof; and
   (e) comparing the detected sizes of the DNA fragments with those of a control;
(21) the method of (17), comprising the steps of:
   (a) preparing a DNA sample from a subject;
   (b) amplifying in the sample the DNA encoding the polypeptide of (5) or the expression control region thereof;
   (c) dissociating the amplified DNA to single-stranded DNAs;
   (d) separating the dissociated single-stranded DNAs on a non-denaturing gel; and
   (e) comparing the mobility of the separated single-stranded DNAs with that of a control;
(22) the method of (17), comprising the steps of:
   (a) preparing a DNA sample from a subject;
   (b) amplifying in the sample the DNA encoding the polypeptide of (5) or the expression control region thereof;
   (c) separating the amplified DNAs on a gel with increasing concentration gradient of a DNA denaturant; and
   (d) comparing the mobilities of the separated DNAs with those of a control;
(23) a method for testing disorders associated with the aberration in the expression of a gene encoding the polypeptide of (5), comprising the step of detecting the expression level of the gene in the subject;
(24) the method of (23) , comprising the steps of:
   (a) preparing an RNA sample from a subject;
   (b) measuring the amount of RNA encoding the polypeptide of (5) contained in said RNA sample; and
   (c) comparing the amount of measured RNA with that of a control;
(25) the method of (23), comprising the steps of:
   (a) providing a cDNA sample prepared from a subject, and a basal plate on which nucleotide probes hybridizing to the DNA encoding the polypeptide of (5) are immobilized;
   (b) contacting said cDNA sample with said basal plate;
   (c) measuring the expressed amount of the gene encoding the polypeptide of (5) contained in said cDNA sample by detecting the hybridization intensity between said cDNA sample and the nucleotide probe immobilized on the basal plate; and
   (d) comparing the measured expression amount of the gene encoding the polypeptide of (5) with that of a control;
(26) the method of (23), comprising the steps of:
   (a) preparing a protein sample from a subject;
   (b) measuring the amount of the polypeptide of (5) contained in said protein sample; and
   (c) comparing the amount of the measured polypeptide with that of a control;
(27) an oligonucleotide having a chain length of at least 15 nucleotides hybridizing to a DNA encoding the polypeptide of (5) or the expression control region thereof;
(28) an assay reagent for testing disorders associated with aberration in the expression of the gene encoding the polypeptide of (5) or aberration in the activity of the polypeptide of (5), comprising the oligonucleotide of (27); and
(29) an assay reagent for testing disorders associated with aberration in the expression of a gene encoding the polypeptide of (5) or aberration in the activity of the polypeptide of (5), comprising the antibody of (7).

In the following, definitions of terms used herein are described to facilitate understanding of the terms used herein, but it should be understood that they are not described so as to limit the present invention in any way.

Herein, the term "guanosine triphosphate-binaing protein coupled receptor (GPCR)" refers to a cytoplasmic membrane receptor that transmits signals into cells via activation of a GTP-binding protein.

The term "polynucleotide" as used herein refers to a ribonucleotide or deoxyribonucleotide or a polymer consisting of a plurality of bases or base pairs. Polynucleotides include single-stranded DNAs as well as double-stranded DNAs. Polynucleotides include both unmodified naturally occurring polynucleotides and modified polynucleotides. Tritylated bases and special bases such as inosine are examples of modified bases.

The term "polypeptide" used herein refers to a polymer comprising a plurality of amino acids. Therefore, oligopeptides and proteins are also included within the concept of polypeptides. Polypeptides include both unmodified naturally occurring polypeptides and modified polypeptides. Examples of polypeptide modifications include acetylation; acylation; ADP-ribosylation; amidation; covalent binding with flavin; covalent binding with heme moieties; covalent binding with nucleotides or nucleotide derivatives; covalent binding with lipids or lipid derivatives; covalent binding with phosphatidylinositols; cross-linkage; cyclization; disulfide bond formation; demethylation; covalent cross linkage formation; cystine formation pyroglutamate formation; formylation; γ-carboxylation; glycosylation; GPI-anchor formation; hydroxylation; iodination; methylation; myristoylation; oxidation; proteolytic treatment; phosphorylation; prenylation; racemization; selenoylation; sulfation; transfer RNA-mediated amino acid addition to a protein such as arginylation; ubiquitination; and the like.

The term "isolation" as used herein refers to a substance (for example, polynucleotide or polypeptide) taken out from the original environment (for example, natural environment for a naturally occurring substance), and "artificially" changed from the natural state. "Isolated" compound refers to compounds comprising compounds present in samples substantially abundant in subject compound and/or those present in samples wherein the subject compound is partly or substantially purified. Herein, the term "substantially purified" refers to compounds (for example, polynucleotides or polypeptides) that are isolated from the natural environment and which do not contain at least 60 %, preferably 75%, and post preferably 90 % of the other components associated with the compound in nature.

The term "mutation" used herein refers to changes of amino acids in an amino acid sequence or changes of bases in a nucleotide sequence (that is, substitution, deletion, addition, or insertion of one or more amino acids or nucleotides). Therefore, the term "mutant" as used herein refers to amino acid sequences wherein one or more amino acid(s) is changed, or nucleotide sequences wherein one or more base(s) is changed. The nucleotide sequence changes in the mutant may either change the amino acid sequence of the polypeptide encoded by the standard polynucleotide or not. The mutant may be one existing in nature, such as an allelic mutant, or one not yet identified in nature. The mutant may be altered conservatively, wherein the substituted amino acid has similar structural or chemical characteristics as that of the original amino acid. Rarely, mutants may be substituted non-conservatively. Guidance to decide which or how many amino acid residues are to be substituted, inserted, or deleted without inhibiting biological or immunological activities can be found using computer programs known in the art, such as the DNA star STAR software.

"Deletion" is a change either in the amino acid sequence or nucleotide sequence, wherein one or more amino acid residues or nucleotide residues are absent, respectively, as compared with the amino acid sequence of a naturally occurring GPCR and GPCR-associated polypeptide, or the nucleotide sequences encoding same.

"Insertion" or "addition" is a change either in the amino acid sequence or nucleotide sequence, wherein one or more amino acid residues or nucleotide residues are added, respectively, as compared with the amino acid sequence of a naturally occurring GPCR and GPCR-associated polypeptide, or nucleotide sequences encoding same.

"Substitution" is a change either in the amino acid sequence or nucleotide sequence, wherein one or more amino acid residues or nucleotide residues are changed for different amino acid residues or nucleotide residues, respectively, as compared with the amino acid sequence of a naturally occurring GPCR and GPCR-associated polypeptide, or nucleotide sequences encoding same.

The term "hybridize" as used herein refers to a process wherein a nucleic acid chain binds to its complementary chain through the formation of base pairs.

In general, the term "treatment" as used herein means to achieve pharmacological and/or physiological effects. Such effects may be either a prophylactic effect, preventing disorders or symptoms completely or partially, or a therapeutic effect curing symptoms of disorders completely or partially. The term "treatment" used herein encompasses all treatments of disorders in mammals, in particular, humans. Moreover, this term also includes prophylaxis of the onset of the disease, suppression of progression of the disorder, and amelioration of the disease in subjects with diathesis of disease who have not been diagnosed as being ill.

The term "ligand" used herein refers to molecules that bind to a polypeptide of the present invention, including both natural and synthetic ligands. "Agonist" refers to molecules that bind and activate a polypeptide of the present invention. On the other hand, "antagonist" refers to molecules that inhibit the activation of a polypeptide of the present invention.

### BRIEF DESCRIPTION OF THE DRAWINGS

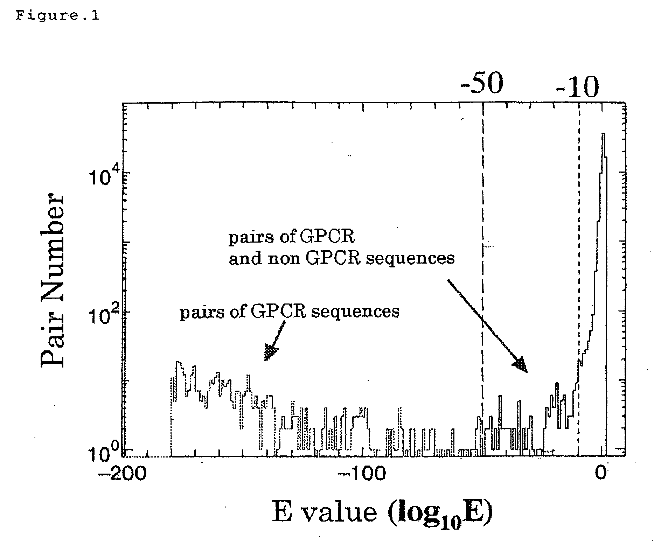

Figure 1 is a graph showing the number of pairs between GPCR sequences and other GPCR sequences or non-GPCR sequences, which were plotted with respect to the E-value, detected during the search of known GPCR sequences in an evaluation data base including 1,054 of GPCR sequences and 64,154 of non-GPCR sequences.

### DETAILED DESCRIPTION OF THE INVENTION

### <Polypeptides>

The present invention provides novel polypeptides belonging to the GPCR family. Nucleotide sequences of 1035 polynucleotides derived from humans, whose sequences have been identified by the present inventors, are shown in the odd-numbered SEQ ID NOs from SEQ ID NO: 1 to SEQ ID NO: 2069. Amino acid sequences of polypeptides encoded by said polynucleotides are shown in the even-numbered SEQ ID NOs from SEQ ID NO: 2 to SEQ ID NO: 2070. In the nucleotide sequences shown in the odd-numbered SEQ ID NOs from SEQ ID NO: 1 to SEQ ID NO: 2069, n means a base selected from a, t, c, and g. In the amino acid sequences shown in the even-numbered SEQ ID NOs from SEQ ID NO: 2 to SEQ ID NO: 2070, Xaa means any one of amino acids. GPCRs have the activity to transmit signals into the cell through the activation of a G protein by the action of a ligand of GPCR, and are associated with genetic diseases and disorders in great many regions of the body, such as the cranial nervous system, the cardiovascular system, the alimentary system, the immune system, the locomotorial system, the urogenital system, etc. Therefore, the polypeptides of this invention can be used to screen for ligands, agonists, or antagonists that control the functions of the polypeptides, which serves as an important target in the development of drugs for above-described disorders.

This invention also provides polypeptides functionally equivalent to the polypeptides identified by the present inventors. Herein, the term "functionally equivalent" means that the subject polypeptide has a biological characteristic equivalent to that of a polypeptide identified by the present inventors. Examples of biological characteristics of GPCRs include: binding activity with a ligand; and the activity to transduce signals into cells through the activation of trimeric GTP-binding proteins. The trimeric GTP-binding proteins are classified into following three categories according to the types of the intracellular signal transduction systems activated thereby: (1) Gq type: elevating the Ca²⁺ level; (2) Gs type: increasing cAMP; and (3) Gi type: suppressing cAMP (Trends Pharmacol. Sci. (99) 20: 118). Therefore, it is possible to assess whether a subjective polypeptide has a biological characteristic equivalent to that of a polypeptide identified by the inventors or not, for example, by detecting the changes in intercellular concentrations of cAMP or calcium caused by the activation.

A method for introducing mutation(s) into the amino acid sequence of a protein can be mentioned as one embodiment of methods for preparing polypeptides functionally equivalent to the polypeptides identified by the inventors. Such a method includes, for example, the site-directed mutagenesis (Current Protocols in Molecular Biology, edit. Ausubel et al. (1987) Publish. John Wiely & Sons Section 8.1-8.5). Amino acid mutation in polypeptides may also occur in nature. The present invention includes mutant proteins, regardless whether artificially or naturally produced, comprising amino acid sequences identified by the inventors (i.e., the even-numbered SEQ ID NOs from SEQ ID No: 2 to SEQ ID NO: 2070), wherein one or more amino acid residues are altered by substitution, deletion, insertion, and/or addition, yet which are functionally equivalent to the polypeptides identified by present inventors.

As for the amino acid residue to be substituted, it is preferable that it be substituted with a different amino acid residue that allows the properties of the amino acid residue to be conserved. For example, Ala, Val, Leu, Ile, Pro, Met, Phe, and Trp are all classified as non-polar amino acids, and are considered to have similar properties to each other. Further, examples of uncharged amino acids are Gly, Ser, Thr, Cys, Tyr, Asn, and-Gin. Moreover, examples of acidic amino acids are Asp and Glu, and those of basic amino acids are Lys, Arg, and His.

There is no limitation in the number and sites of the amino acid mutation in these polypeptides so long as the mutated polypeptide retains the functions of the original polypeptide. The number of mutations may be typically less than 10%, preferably less than 5%, and more preferably less than 1% of the total amino acid residues.

Other embodiments of the method for preparing polypeptides functionally equivalent to the polypeptides identified by the inventors include methods utilizing hybridization techniques or gene amplification techniques. More specifically, those skilled in the art can obtain polypeptides functionally equivalent to the polypeptides determined by the present inventors by isolating highly homologous DNAs from DNA samples derived from organisms of the same or different species using hybridization techniques (Current Protocols in Molecular Biology, edit. Ausubel et al. (1987) Publish. John Wiley & Sons Section 6.3-6.4) based on the DNA sequences encoding the polypeptides identified by the inventors (i.e., sequences of odd-numbered SEQ ID NOs from SEQ ID NO: 1 to SEQ ID NO: 2069). Thus, such polypeptides encoded by DNAs hybridizing to the DNAs encoding the polypeptides identified by the inventors, which polypeptides are functionally equivalent to the polypeptides identified by the inventors, are also included in the polypeptides of this invention.

Examples of organisms to be used for isolating such polypeptides are rats, mice., rabbits, chicken, pigs, cattle, etc., as well as humans, but the present invention is not limited to these organisms.

The hybridization stringency required to isolate a DNA encoding a functionally equivalent polypeptide to the polypeptides identified by the inventors is normally "1x SSC, 0.1% SDS, 37°C" or so, a more stringent condition being "0.5x SSC, 0.1% SDS, 42°C" or so, and a much more stringent condition being "0.2x SSC, 0.1% SDS, 65°C" or so. As the stringency becomes higher, isolation of a DNA with higher homology to the probe sequence can be expected. However, above-mentioned combinations of conditions of SSC, SDS, and temperature are only an example, and those skilled in the art can achieve the same stringency as described above by appropriately combining above-mentioned factors or others parameters which determine the stringency of the hybridization (for example, probe concentration, probe length, reaction time of hybridization, etc.).

The polypeptides encoded by the DNA isolated using such hybridization techniques normally are highly homologous in their amino acid sequences to the polypeptides identified by the present inventors. Herein, high homology indicates a sequence identity of at least 40% or more, preferably 60% or more, more preferably 80% or more, still more preferably 90% or more, further still more preferably at least 95% or more, and yet more preferably at least 97% or more (for example, 98% to 99%). Homology of amino acid sequences can be determined, for example, by using the algorithm BLAST according to Karlin and Altschul (Proc. Natl. Acad. Sci. USA 87: 2264-2268 (1990); Proc. Natl. Acad. Sci. USA 90: 5873-5877 (1993)). Based on this algorithm, a program referred to as BLASTX has been developed (Altschul et al., J. Mol. Biol. 215: 403-410 (1990)). When amino acid sequences are analyzed using BLASTX, parameters are set, for example, score = 50 and wordlength = 3, while in the case of using BLAST and Gapped BLAST programs, default parameters of each program are used. Specific techniques of these analytical methods are well known in the field (See http://www.ncbi.nlm.nih.gov.).

The gene amplification technique (PCR) (Current Protocols in Molecular Biology, edit. Ausubel et al. (1987) Publish. John Wiley & Sons Section 6.1-6.4) can be utilized to obtain a polypeptide functionally equivalent to the polypeptides isolated by the present inventors, based on DNA fragments isolated as highly homologous DNAs to the DNA sequences encoding the polypeptides isolated by the present inventors, by designing primers based on a part of the DNA sequences encoding the polypeptides identified by the inventors (sequences of odd-numbered SEQ ID NOs from SEQ ID NO: 1 to SEQ ID NO: 2069).

Polypeptides of this invention may be in the form of a "mature" protein, or may be also a part of a larger protein, such as fusion proteins. Polypeptides of this invention may contain secretory sequences, namely leader sequences; prosequences; sequences useful for purification, such as multiple histidine residues and such; and additive sequences to secure the stability during recombinant production.

### <Polypeptide fragments>

The present invention also provides fragments of the polypeptides of this invention. These fragments are polypeptides having amino acid sequences which are partly, but not entirely, identical to the above polypeptides of this invention. The polypeptide fragments of this invention usually consist of 8 amino acid residues or more, and preferably 12 amino acid residues or more (for example, 15 amino acid residues or more). Examples of preferred fragments include truncation polypeptides, having amino acid sequences lacking a series of amino acid residues including either the amino terminus or carboxyl terminus, or two series of amino acid residues, one including the amino terminus and the other including the carboxyl terminus. Furthermore, fragments featured by structural or functional characteristics are also preferable, which include those having α-helix and α-helix forming regions, β-sheet and β-sheet forming regions, turn and turn forming regions, coil and coil forming regions, hydrophilic regions, hydrophobic regions, α-amphipathic regions, β-amphipathic regions, variable regions, surface forming regions, substrate-binding regions, and high antigenicity index region. Biologically active fragments are also preferred. Biologically active fragments mediate the activities of the polypeptides of this invention, which fragments include those having similar or improved activities, or reduced undesirable activities. For example, fragments having the activity to transduce signals into cells via binding of a ligand, and furthermore, fragments having antigenicity or immunogenicity in animals, especially humans are included. These polypeptide fragments preferably retain the biological activities of the polypeptides of this invention, which activity includes antigenicity. Mutants of specific sequences or fragments also constitute an aspect of this invention. Preferred mutants are those which are different from the subject polypeptide, due to replacement with conservative amino acids, namely, those in which residue(s) is (are) substituted with other residue (s) having similar properties. Typical substitutions are those between Ala, Val, Leu, and Ile; Ser and Thr; acidic residues Asp and Glu, Asn, and Gln; basic residues Lys and Arg; or aromatic residues Phe and Tyr.

Alternatively, fragments which bind to ligands without transducing signals into cells may be also useful as competitive inhibitors for the polypeptides of this invention and are included in the present invention.

### <Production of polypeptides>

Polypeptides of this invention may be produced by any appropriate method. Such polypeptides include isolated naturally-occurring polypeptides, and polypeptides which are produced by gene recombination, synthesis, or by a combination thereof. Procedures for producing these polypeptides are well known in the art. Recombinant polypeptides may be prepared, for example, by transferring a vector, wherein the polynucleotide of the present invention is inserted, into an appropriate host cell, and purifying the polypeptide expressed within the resulting transformant. On the other hand, naturally occurring polypeptides can be prepared, for example, using affinity columns, wherein antibodies against the polypeptide of this invention (described below) are immobilized (Current Protocols in Molecular Biology, edit. Ausubel et al. (1987) Publish. John Wiley & Sons Section 16.1-16.19). Antibodies for affinity purification may be either polyclonal or monoclonal antibodies- The polypeptides of this invention may be also prepared by the *in vitro* translation method (for example, see "On the fidelity of mRNA translation in the nuclease-treated rabbit reticulocyte lysate system." Dasso, M. C. and Jackson, R. J. (1989) NAR 17: 3129-3144), and so on. Polypeptide fragments of this invention can be produced, for example, by cleaving the polypeptides of the present invention with appropriate peptidases.

### <Polynucleotides>

The present invention also provides polynucleotides encoding the polypeptides of this invention. The polynucleotides of this invention include: those encoding polypeptides comprising the amino acid sequences of even-numbered SEQ ID NOs from SEQ ID NO; 2 to SEQ ID NO: 2070; those comprising the coding regions of the nucleotide sequences of odd-numbered SEQ ID NOs from SEQ ID NO: 1 to SEQ ID NO: 2069; and those comprising different nucleotide sequences from those of odd-numbered SEQ ID NOs from SEQ ID NO: 1 to SEQ ID NO: 2069 due to the degeneracy of genetic codes but still encoding polypeptides comprising amino acid sequences of even-numbered SEQ ID NOs from SEQ ID NO: 2 to SEQ ID NO: 2070. Furthermore, the polynucleotides of this invention include those encoding polypeptides functionally equivalent to the polypeptides of the present invention, comprising nucleotide sequences which are homologous to said polynucleotide sequences at least 40% or more, preferably 60% or more, more preferably 80% or more, further more preferably 90% or more, and still preferably 95% or more, and further still more preferably 97% or more (for example, 98% to 99%) in the entire length. Homology of the nucleotide sequences can be determined, for example, using the BLAST algorithm by Karlin and Altschul (Proc. Natl. Acad. Sci. USA 87; 2264-2268 (1990) ; Proc. Natl. Acad. Sci. USA 90: 5873-5877 (1993)). Based on this algorithm, an algorithm called BLASTN has been developed (Altschul et al. J. Mol. Biol. 215: 403-410 (1990)). When analyzing a nucleotide sequence using the BLASTN program, parameters are set, for example, score = 100 and wordlength = 12. When using both BLAST and Gapped BLAST programs, default parameters of each program are used. Specific techniques of these analytical methods are well known in the art (http://www.ncbi.nlm.nih.gov.). The polynucleotides of this invention also include polynucleotides having a nucleotide sequences complementary to those of the above-described polynucleotides.

The polynucleotides of this invention can be obtained for example, from cDNA libraries induced from intracellular mRNAs by standard cloning and screening methods- Moreover, the polynucleotides of this invention can be obtained from natural sources, such as genomic libraries, and also can be synthesized using commercially available techniques known in the art.

Polynucleotides comprising nucleotide sequences significantly homologous to the polynucleotide sequences identified by the inventors (sequences of odd-numbered SEQ ID NOs from SEQ ID NO: 1 to SEQ ID NO: 2069) can be prepared using, for example, hybridization techniques (Current Protocols in Molecular Biology, edit. Ausubel et al. (1987) Publish. John Wiley & Sons Section 6.3-6.4) and the gene amplification technique (PCR) (Current Protocols in Molecular Biology, edit. Ausubel et al. (1987) Publish. John Wiley & Sons Section 6.1-6.4). That is, based on the polynucleotide sequences identified by the inventors (sequences of odd-numbered SEQ ID NOs from SEQ ID NO: 1 to SEQ ID NO: 2069) or portions thereof, using hybridization techniques, DNAs highly homologous to these polynucleotides can be isolated from DNA samples derived from the same or different species of organisms. Moreover, polynucleotides highly homologous to the sequences of said polynucleotides can be isolated using the gene amplification technique by designing primers based on portions of the polynucleotide sequences identified by the inventors (sequences of odd-numbered SEQ ID NOs from SEQ ID NO: 1 to SEQ ID NO: 2069). Therefore, the present invention includes polynucleotides hybridizing under stringent conditions to the polynucleotides comprising the nucleotide sequences of odd-numbered SEQ ID NOs from SEQ ID NO: 1 to SEQ ID NO: 2069. The conditions for stringent hybridization are usually "1x SSC, 0.1% SDS, 37°C" or so, with a more stringent condition being "0.5x SSC, 0.1% SDS, 42°C" or so, and a furthermore stringent one being "0.2x SSC, 0.1% SDS, 65°C" or so. The more stringent the hybridization conditions are, the more highly homologous DNAs to the probe sequence can be expected. However, the above-described combinations of conditions of SSC, SDS, and temperature are mere examples, and those skilled in the art may achieve similar stringency as described above by appropriately combining the aforementioned factors or others parameters that determine the hybridization stringency (for example, probe concentration, probe length, reaction time of hybridization, etc.).

Polynucleotides comprising nucleotide sequences significantly homologous to the sequences of the polyncleotides identified by the inventors can also be prepared by inducing mutations into the nucleotide sequences of odd-numbered SEQ ID NOs from SEQ ID NO: 1 to SEQ ID NO: 2069 (for example, the site-directed mutagenesis) (Current Protocols in Molecular Biology, edit. Ausubel, et al. (1987) Publish. John Wiley & Sons Section 8.1-8.5). Such polynucleotides may be also generated by mutation in nature. The present invention includes polynucleotides encoding polypeptides comprising amino acid sequences of even-numbered SEQ ID NOs from SEQ ID NO: 2 to SEQ ID NO: 2070 wherein one or more amino acid residues are substituted, deleted, inserted, and/or added, due to such mutations of the nucleotide sequences.

Polynucleotides used for recombinant production of the polypeptide of this invention include the coding sequences of the mature polypeptide or fragments thereof alone; and coding sequences of the mature polypeptide or fragments thereof in the same reading frame with other coding sequences (for example, leader or secretory sequences; pre-, pro-, or preproprotein sequences; or sequence encoding other fusion peptide portions). For example, a marker sequence that facilitates purification of the fusion polypeptide may be encoded in the same reading frame. A preferred embodiment of this invention includes specific marker sequences, such as the hexahistidine peptide or Myc tag provided by the pcDNA3.1/Myc-His vector (Invitrogen), which is described in the literature (Gentz et al., Proc. Natl. Acad Sci. USA (1989) 86: 821-824). Further, this polynucleotide may comprise a 5'- and 3'-noncoding sequence, for example, transcribed but non-translated sequences; splicing and polyadenylation signals; ribosome-binding sites; and mRNA stabilization sequences.

### <Probe, Primer, Antisense, Ribozyme>

The present invention provides nucleotides, having a chain length of at least 15 nucleotides, which are complementary to a polynucleotide isolated by the present inventors (a polynucleotide or a complementary strand thereof consisting of the nucleotide sequences of odd-numbered SEQ ID NOs from SEQ ID NO: 1 to SEQ ID NO: 2069). Herein, the term "complementary strand" is defined as one strand of a double strand nucleic acid composed of A:T (A:U in case of RNA) and G:C base pairs to the other strand. Also, "complementary" is defined as not only those completely matching within a continuous region of at least 15 sequential nucleotides, but also those having a homology of at least 70%, preferably at least 80%, more preferably 90%, and most preferably 95% or higher within that region. The homology may be determined using the algorithm described herein. Probe and primers for detection or amplification of the polynucleotides of the present invention are included in these polynucleotides. Typical polynucleotides used as primers have a chain length of 15 to 100 nucleotides, and preferably 15 to 35 nucleotides. Alternatively, polynucleotides used as probes are nucleotides having a chain length of at least 15 nucleotides, preferably at least 30 nucleotides, containing at least a portion or the whole sequence of a DNA of the present invention. Such nucleotides preferably hybridize specifically to a DNA encoding a polypeptide of the present invention. The term "hybridize specifically" defines that it hybridizes under a normal hybridization condition, preferably a stringent condition with a nucleotide identified by the present inventors (sequence shown as odd-numbered SEQ ID NOs from SEQ ID NO: 1 to SEQ ID NO: 2069), but not with DNAs encoding other polypeptides.

These nucleotides can be used for detecting and diagnosing abnormal activities of the polypeptides of the present invention or abnormal expression of genes encoding the polypeptides.

Further, these nucleotides include polynucleotides that suppress the expression of genes encoding the polypeptides of the present invention. Such polynucleotides include antisense DNAs (DNAs encoding antisense RNAs, which are complementary to transcriptional products of the genes encoding the polypeptides of the present invention) and ribozymes (DNAs encoding RNAs having ribozyme activities to specifically cleave transcriptional products of the genes encoding the polypeptides of the present invention).

A plurality of factors, such as those described below, arise as a result of actions suppressing the expression of a target gene by an antisense DNA: inhibition of the transcription initiation by the formation of a triple strand; suppression of the transcription through hybridization with a local open loop conformation site formed by an RNA polymerase; inhibition of the transcription by hybridization with RNA, which is in course of synthesis; suppression of the splicing through hybridization at a junction of intron and exon; suppression of the splicing through hybridization with a spliceosome forming site; suppression of the transfer from the nuclei to cytoplasm through hybridization with the mRNAs; suppression of the splicing through hybridization with capping sites or poly(A) addition sites; suppression of the translation initiation through hybridization with a translation initiation factor binding site; suppression of the translation through hybridization with the ribosome binding site near the initiation codon; inhibition of the elongation of the peptide chain through hybridization with the translation regions and polysome binding sites of the mRNAs; suppression of the expression of genes by hybridization with the interaction sites between nucleic acids and proteins; and the like. These actions inhibit the processes of transcription, splicing, and/or translation to suppress the expression of a target gene (Hirajima and Inoue, "New Biochemistry Experimental Course No. 2, Nucleic Acid IV, Duplication and Expression of Genes", Japan Biochemical Society ed., Tokyo Kagaku Doujin, pp. 319-347 (1993)).

The antisense DNA of the present invention may suppress the expression of the target gene through any of the above-mentioned actions. According to one embodiment, an antisense sequence designed to be complementary to a non-translated region near the 5'-terminus of mRNA of a gene may effectively inhibit the translation of the gene. Additionally, sequences which are complementary to the coding region or the 3' non-translated region can be also used. As described above, DNA containing antisense sequences not only to the translation region of a gene, but also those to sequences of non-translated regions are included in the antisense DNA of the present invention. The antisense DNAs to be used in the present invention are linked to downstream of an appropriate promoter, and a sequence including a transcriptional termination signal is preferably linked to the 3'-side thereof. The sequence of the antisense DNA is preferably complementary to the target gene or a part thereof; however, so long as the expression of the gene can be effectively inhibited, it does not have to be a completely complementary DNA. The transcribed RNA is preferably 90% or more, more preferably 95% or more, complementary to the transcribed product of the target gene. In order to effectively inhibit the expression of the target gene using an antisense sequence, the antisense DNA has at least a chain length of 15 bp or more, preferably 100 bp, more preferably 500 bp, and usually has a chain length less than 3000 bp, preferably less than 2000 bp to cause an antisense effect.

Such antisense DNA can be also applied to gene therapy for diseases caused by abnormalities (functional abnormalities or expression abnormalities) of the polypeptides of the present invention, and the like. The antisense DNA can be prepared by, for example, the phosphorothionate method (Stein, "Physicochemical properties of phosphorothioate oligodeoxynucleotides." Nucleic Acids Res. 16, 3209-21 (1988)) and the like based on the sequence information of a DNA (for example, sequences of odd-numbered SEQ ID NOs from SEQ ID NO: 1 to SEQ ID NO: 2069)) encoding a polypeptide of the present invention.

Further, suppression of the expression of endogenous genes can be also achieved utilizing DNAs encoding ribozymes. Ribozymes are RNA molecules having catalytic activity. There exist ribozymes having various activities, and the research of ribozymes as an enzyme for truncating RNA allowed for the design of ribozymes that cleave RNAs in a site-specific manner. There are ribozymes which are larger than 400 nucleotides, such as Group I intron type ribozymes, and M1RNA comprised in RNaseP, and those which have an active domain of about 40 nucleotides, called hammer-head type and a hairpin type ribozymes (Makoto Koizumi and Eiko Ohtsuka, (1990) , Protein Nucleic Acid and Enzyme (PNE) 35:2191).

For example, the hammer head type ribozyme cleaves the 3'-side of C15 of G13U14C15 within its own sequence. A base pair formation of the U14 with the A at position 9 is important for the activity, and it is shown that the clevage proceeds even if the C at position 15 is A or U (M. Koizumi et al., (1988) FEBS Lett. 228:225). Restriction enzymatic RNA-truncating ribozymes recognizing sequences of UC, UU, and UA in a target RNA may be generated by designing the substrate binding site of the ribozyme complementary with the RNA sequence near the target site (M. Koizumi, et al., (1988) FEBS Lett. 239:285; Makoto Koizumi and Eiko Ohtsuka, (1990), Protein Nucleic Acid and Enzyme (PNE) 35:2191); and M. Koizumi et al. (1989), Nucleic Acids Res. 17:7059). A plurality of sites, which can be used as a target, exist among the polynucleotides (having sequence of odd-numbered SEQ ID NOs from SEQ ID NO: 1- to SEQ ID NO: 2069) identified by the present inventors.

Further, the hairpin type ribozymes are also useful in the context of the present invention. The hairpin type ribozymes are found on, for example, the minus chain of a satellite RNA of tobacco ringspot virus (J. M. Buzayan, Nature 323:349 (1986)). It is also demonstrated that the ribozyme can be designed to cause a target specific RNA truncation (Y. Kikuchi and N. Sasaki, (1992) Nucleic Acids Res. 19:6751; and Y. Kikuchi, (1992) Chemistry and Organism 30:112).

When the polynucleotides suppressing the expression of the genes encoding the polypeptides of the present invention are used in gene therapy, they may be administered to a patient by the ex *vivo* method, *in vivo* method, and the like, using, for example, viral vectors such as retroviral vector, adenoviral vector, adeno-associated viral vectors, and such; and non-viral vectors such as liposome; and so on.

### <Production of Vector, Host cell, and Polypeptide>

Further, the present invention provides methods for producing vectors containing a polynucleotide of the present invention, host cells retaining a polynucleotide of the present invention or said vector, and polypeptides of the present invention utilizing said host cells.

The vector of the present invention is not limited so long as the DNA inserted in the vector is retained stably. For example, pBluescript vector (Stratagene) is preferable as a cloning vector when using *E. coli* as the host. When the vector is used for producing a polypeptide of the present invention, an expression vector is particularly useful. The expression vector is not specifically limited so long as it expresses polypeptides *in vitro,* in *E. coli*, in cultured cells, and *in vivo.* However, preferable examples include the pBEST vector (ProMega) for *in vitro* expression, the pET vector (Invitrogen) for expression in *E. coli,* the pME18S-FL3 vector (GenBank Accession No. AB009864) for the expression in cultured cells, and the pME18S vector (Mol. Cell Biol. 8:466-472(1988)) for in *vitro* expression, and so on. The insertion of a DNA of the present invention into a vector can be carried out by conventional methods, for example, by the ligase reaction using restriction enzyme sites (Current Protocols in Molecular Biology, edit. Ausubel, et al., (1987) Publish. John Wiley & Sons, Section 11.4-11.11).

The host cell to which the vector of the present invention is introduced is not specifically limited, and various host cells can be used according to the objects of the present invention. For example, bacterial cells (e.g. *Streptococcus, Staphylococcus, E.* coli, Streptomyces, Bacillus subtilis), fungal cells (e.g. yeast, Aspergillus), insect cells (e.g. Drosophila 52, Spodoptera SF9), animal cells (e.g. CHO, COS, HeLa, C127, 3T3, BHK, HEK293, Bowes melanoma cell), and plant cells can be exemplified as cells to express polypeptides. The transfection of a vector to a host cell can be carried out by conventional methods, such as the calcium phosphate precipitation method, - the electroporation method (Current protocols in Molecular Biology, edit., Ausubel et al., (1987) Publish. John Wiley & Sons, Section 9.1-9.9), the Lipofectamine method (GIBCO-BRL), the microinjection method, and so on.

Appropriate secretion signals can be incorporated into the polypeptide of interest in order to secrete polypeptides into the lumen of endoplasmic reticulum, into cavity around the cell, or into the extracellular environment by expressing them in a host cell. These signals may be endogenous signals or signals from a different species to the objective polypeptide.

When a polypeptide of the present invention is secreted into the culture media, the culture media is collected to collect the polypeptide of the present invention. When a polypeptide of the present invention is produced intracellularly, the cells are first lysed, and then, the polypeptides are collected.

In order to collect and purify a polypeptide of the present invention from a recombinant cell culture, methods known in the art including ammonium sulfate or ethanol precipitation, extraction by acid, anionic or cationic exchange chromatography, phosphocellulose chromatography, hydrophobic interaction chromatography, affinity chromatography, hydroxylapatite chromatography, and lectin chromatography can be used.

### <Test method>

The present invention provides a method for testing diseases related to abnormal expression of the genes encoding the polypeptides of the present invention, or abnormal activities of the polypeptides of the present invention. It is considered that GPCR has an important function *in vivo*, and thus, abnormal expression and function thereof may cause various diseases. Therefore, assay of diseases may be accomplished using inappropriate activities or expression of the polypeptides of the present invention as an index.

The term "assay of diseases" includes not only tests to draft therapeutic strategy for a subject who exhibits the symptom of a disease, but also tests for preventing diseases by determining whether the subject is susceptible- to the disease.

One embodiment of the test methods of the present invention is a method comprising the step of detecting a mutation in a gene encoding a polypeptide of the present invention or in the expression control regions thereof in a subject.

More specifically, the test can be accomplished by directly determining the nucleotide sequence of a gene encoding a polypeptide of the present invention or its expression control region in a subject. According to this method, first, a DNA sample is prepared from a subject. The DNA sample can be prepared from chromosomal DNA or RNA extracted from cells of the subject, for example, the biopsy or autopsy specimen of blood, urine, saliva, and tissue. In order to prepare a DNA sample for the present method from a chromosomal DNA, a genomic library may be produced by, for example, digesting the chromosomal DNA with appropriate restriction enzymes, and then cloning the digested DNA to a vector. On the other hand, for example, a cDNA library may be prepared from RNA by using reverse transcriptase to prepare a DNA sample for the present method from RNA. Next, DNA containing a gene encoding a polypeptide of the present invention or the expression control region thereof is isolated according to the present method. The isolation of a DNA can be carried out by screening the genomic library or cDNA library using probes hybridizing with the DNA containing the gene encoding. the polypeptide of the present invention or its expression control region. The isolation of a DNA can be also carried out by PCR using the genomic DNA library, cDNA library, and RNA as the template, and primers hybridizing to a DNA containing a gene encoding a polypeptide of the present invention or its expression control region. Then, the nucleotide sequence of the isolated DNA is determined according to the present method. The determination of the nucleotide sequence of selected DNAs can be carried out by methods known to those skilled in the art. According to the present method, the determined nucleotide sequence of the DNA is then compared with that of a control. The "control" herein refers to a nucleotide sequence of DNAs containing a gene encoding a normal (wild type) polypeptide of the present invention or its expression control region. When the nucleotide sequence of a DNA of a subject differs from those of the control as a result of a comparison above, the subject is judged to be afflicted with disease or in danger of the onset of disease.

According to the test method of the present invention, various methods can be used other than the method directly determining the nucleotide sequence of a DNA, which was derived from the subject, as described above.

In one embodiment of the method, a DNA sample is first prepared from a subject and is digested with restriction enzymes. Then, the DNA fragments are separated in accordance with their size, followed by comparison of the detected sizes of the DNA fragments with those of a control. Alternatively, in another embodiment, a DNA sample is first prepared from a subject. Then, DNA containing a gene encoding a polypeptide of the present invention or its expression control region is amplified from the sample, and the amplified DNAs are digested with restriction enzymes. After separating the DNA fragments according to their size, the detected sizes of the DNA fragments are compared with those of a control.

Such methods include, for example, a method utilizing the Restriction Fragment Length Polymorphism/RFLP, the PCR-RFLP method, and the like. Specifically, when variations exist for the recognition sites of a restriction enzyme, or when insertion (s) or deletion (s) of base(s) exists in a DNA fragment generated by a restriction enzyme treatment, the sizes of fragments that are generated after the restriction enzyme treatment vary in comparison with those of a control. The portion containing the mutation is amplified by PCR, and then, is treated with respective restriction enzymes to detect these mutations as a difference of the mobility of bands after electrophoresis. Alternatively, the presence or absence of the mutations can be detected by carrying out the Southern blotting with a probe DNA of the present invention after treating the chromosomal DNA with respective restriction enzymes followed by electrophoresis. The restriction enzymes to be used can be appropriately selected in accordance with respective mutations. The Southern blotting can be conducted not only on the genomic DNA but also on cDNAs directly digested with restriction enzymes, wherein the cDNAs are converted by the use of a reverse transcriptase from RNAs prepared from subjects. Alternatively, after amplifying DNAs containing a gene encoding a polypeptide of the present invention or its expression control region by PCR using the cDNA as a template, the cDNAs are digested with restriction enzymes and the difference of mobility on an electrophoresis gel of DNA fragments generated by the digestion are examined.

In another embodiment of the present method, a DNA sample is first prepared from a subject. Then, a DNA containing a gene encoding a polypeptide of the present invention or its expression control region is amplified. Thereafter, the amplified DNA is dissociated into single strand DNAs, and the single strand DNAs are separated on a non-denaturing gel. The mobility of the separated single strand DNAs on the gel is compared with those of a control.

Such methods include, for example, the PCR-SSCP (single-strand conformation polymorphism) method ("Cloning and polymerase chain reaction-single-strand conformation polymorphism analysis of anonymous Alu repeats on chromosome 11." Genomics. 1992, Jan. 1, 12(1): 139-146; "Detection of p53 gene mutations in human brain tumors by single-strand conformation polymorphism analysis of polymerase chain reaction products." Oncogene. 1991, Aug. 1; 6(8): 1313-1318; "Multiple fluorescence-based PCR-SSCP analysis with postlabelling." PCR Methods Appl. 1995, Apr. 1; 4(5) : 275-282). This method is particularly preferable for screening many DNA samples, since it has advantages such as: comparative simplicity of operation; small amount of a test sample required; and so on. The principle of the method is as follows. A single strand DNA dissociated from a double-strand DNA fragment forms a unique higher conformation depending on respective nucleotide sequence. Complementary single-stranded DNAs having the same chain length of the dissociated DNA strand shift to different positions in accordance with the difference of the respective higher conformations after electrophoresis on a polyacrylamide gel without a denaturant. The higher conformation of a single-stranded DNA changes even by a substitution of one base, which change results in a different mobility by polyacrylamide gel electrophoresis. Accordingly, the presence of a mutation in a DNA fragment due to point mutation, deletion, insertion, and such can be detected by detecting the change of the mobility.

More specifically, DNA containing a gene encoding a polypeptide of the present invention (or its expression control region) is first amplified by PCR and the like. Preferably, a DNA of a length of about 200 bp to 400 bp is amplified. Those skilled in the art can appropriately select the condition and such for the PCR. DNA products amplified by PCR can be labeled by primers, which are labeled with isotopes such as ³²P; fluorescent dyes; biotin; and so on. Alternatively, the amplified DNA products can be also labeled by conducting PCR in a reaction solution containing substrate bases, which are labeled with isotopes such as ³²P; fluorescent dyes; biotin; and so on. Further, the labeling can be also carried out by adding substrate bases, which are labeled with isotope such as ³²P; fluorescent dyes; biotin; and so on, to the amplified DNA fragment using Klenow enzyme and such, after the PCR reaction. Then, the obtained labeled DNA fragments are denatured by heating and the like, and electrophoresis is carried out on a polyacrylamide gel without a denaturant such as urea. The condition for the separation of the DNA fragments by this electrophoresis can be improved by adding appropriate amounts (about 5% to 10%) of glycerol to the polyacrylamide gel. Further, although the condition for electrophoresis varies depending on the property of respective DNA fragments, it is usually carried out at room temperature (20°C to 25°C). When a preferable separation is not achieved at this temperature, a temperature at which optimum mobility can be achieved is searched from 4°C to 30°C. The mobility of the DNA fragments is detected by autoradiography with X-ray films, scanner for detecting fluorescence, and the like, after the electrophoresis to analyze the result. When a band with different mobility is detected, the presence of a mutation can be confirmed by directly excising the band from the gel, amplifying it again by PCR, and directly sequencing the amplified fragment. Further, the bands can be also detected by staining the gel after electrophoresis with ethidium bromide, silver, and such, without using labeled DNAs.

In still another method, a DNA sample is first prepared from a subject. DNA containing a gene encoding a polypeptide of the present invention or its expression control region is amplified, and then, the amplified DNAs are separated on a gel with gradient concentration of a DNA denaturant. The mobilities of the separated DNAs on the gel are compared with those of a control.

For example, the denaturant gradient gel electrophoresis method (DGGE method) and the like can be exemplified as such methods. The DGGE method comprises the steps of: (1) electrophoresing the mixture of DNA fragments on a polyacrylamide gel with gradient concentration of denaturant; and (2) separating the DNA fragments in accordance with the difference of instabilities of respective fragments. Unstable DNA fragments containing mismatches dissociated partly to a single-strand near the mismatches because of the instability of the DNA sequence by shifting to a part with a certain concentration of the denaturant on the gel. The mobility of the partly-dissociated DNA fragment becomes remarkably slow, ending in a difference of the mobility with that of perfectly double-stranded DNAs without dissociated parts, which allows separation of these DNAs. Specifically, DNA containing a gene encoding a polypeptide of the present invention or its expression control region is (1) amplified by PCR and the like with a primer of the present invention and such; (2) electrophoresed on a polyacrylamide gel with gradient concentration of denaturant such as urea; and (3) the result is compared with that of a control. The presence or absence of a mutation can be detected by detecting the difference of mobility of the DNA fragment due to the extreme slowing down of the mobility speed of the fragment by separation into single-stranded DNAs of a DNA fragment with mutations at parts of the gel where the concentration of the denaturant is lower.

In addition to the above-mentioned methods, the Allele Specific Oligonucleotide (ASO) hybridization method can be used to detect mutations at only specific sites. An oligonucleotide with a nucleotide sequences contained to have a mutation is prepared, and is subjected to hybridization with a DNA sample. The efficiency of hybridization is reduced by the-existence of a mutation. The decrease can be detected by the Southern blotting method; methods which utilize a specific fluorescent reagent that have a characteristic to quench by intercalation into the gap of the hybrid; and the like. Further, the detection may be also conducted by the ribonuclease A mismatch truncation method. Specifically, DNA containing a gene encoding a polypeptide of the present invention is amplified by PCR and the like, and the amplified DNAs are hybridized with labeled RNAs, which were prepared from a control cDNA and the like to incorporate them into a plasmid vector and the like. The presence of a mutation can be detected with autoradiography and the like, after cleaving those sites that form a single-stranded conformation due to the existence of a mutation with ribonuclease A.

Another embodiment of the test method of the present invention is a method comprising the step of detecting the expression level of a gene encoding a polypeptide of the present invention. Herein, transcription and translation are included in the meaning of the term "expression of a gene". Accordingly, mRNAs and proteins are included in the term "expression product".

First, an RNA sample is prepared from a subject according to the method for testing the transcription level of a gene encoding a polypeptide of the present invention. Then, the amount of RNA encoding the polypeptide of the present invention in the RNA sample is measured. Thereafter, the measured amount of the RNA encoding the polypeptide of the invention is compared with that of a control.

A Northern blotting method using a probe which hybridizes with the polynucleotide encoding a polypeptide of the present invention; an RT-PCR method using a primer which hybridizes with a polynucleotide encoding the polypeptide of the present invention; and such can be exemplified as such methods.

Further, a DNA array (Masami Muramatsu and Masashi Yamamoto, New Genetic Engineering Handbook pp. 280-284, YODOSHA Co., LTD.) can also be utilized in the test for the transcription level of the gene encoding the polypeptide of the present invention. Specifically, first, a cDNA sample prepared from a subject and a basal plate on which polynucleotide probes hybridizing with the polynucleotides encoding the polypeptides of the present invention are fixed are provided. Plural kinds of polynucleotide probes can be fixed on the basal plate in order to detect plural kinds of polynucleotides encoding the polypeptides of the present invention. Preparation of a cDNA sample from a subject can be carried out by methods well known to those skilled in the art. In a preferable embodiment for the preparation of the cDNA sample, first, total RNAs are extracted from a cell of a subject. Example of cells include cells of the biopsy or autopsy specimen, of blood, urine, saliva, tissue, and the like. The extraction of total RNAs can be carried out, for example, as follows. So long as total RNAs with high purity can be prepared, known methods, kits, and such can be used. For example, total RNAs are extracted by using "Isogen" (Nippon Gene) following a pretreatment with "RNA later" (Ambion). Specific procedures of the method may be carried out according to the attached protocol. Then, the cDNA sample is prepared by synthesizing cDNAs with reverse transcriptase using extracted total RNAs as a template. The synthesis of cDNA from total RNAs can be carried out by conventional methods known in the art. The prepared cDNA sample is labeled for detection according to needs. The labeling substance is not specifically limited so long as it can be detected, and include, for example, fluorescent substances, radioactive elements, and so on. The labeling can be carried out by conventional methods (L. Luo et al., "Gene expression profiles of laser-captured adjacent neuronal subtypes", Nat. Med. (1999) pp. 117-122).

The term "basal plate" herein refers to a board type material on which polynucleotides can be fixed. So long as polynucleotides can be immobilized on the plate, there is no restriction on the basal plate of the present invention. However, a basal plate that is generally used in the DNA array technique is preferred.

An advantage of the DNA array technique is that the amount of solution needed for hybridization is very small, and that extremely complicated targets containing cDNA derived from the total RNAs of a cell can be hybridized to the fixed nucleotide probes. In general, a DNA array comprises thousands of nucleotides which are printed on a basal plate at a high density. Usually, DNAs are printed on the surface layer of a non-porous basal plate. The surface layer of the basal plate is usually glass, but a porous film, for example, such as nitrocellulose membrane, can be also used. There are two types for fixation (array) of the nucleotides: one is the array based on polynucleotides developed by Affymetrix Co., Ltd.; and the other is the array of cDNA mainly developed by Stanford University. The polynucleotides are usually synthesized *in situ* for the array of the polynucleotide. For example, *in situ* synthesis method of polynucleotides such as the photolithographic technique (Affymetrix) ; and the ink-jet technique (Rosetta Inpharmatics) for fixing a chemical substance; and so on are already known in the art, - and any of these techniques can be used for the production of basal plates of the present invention. There is no limitation on the polynucleotide probes to be fixed on the basal plates, so long as it specifically hybridizes with a gene encoding a polypeptide of the present invention. The polynucleotide probe of the present invention includes polynucleotides and cDNAs. Herein, the term "specifically hybridizes" means that a polynucleotide substantially hybridizes with a polynucleotide encoding a-polypeptide of the present invention and substantially does not hybridize with other polynucleotides. So long as specific hybridization is possible, the polynucleotide probe does not have to be completely complementary to the nucleotide sequence to be detected. Generally, to immobilize a cDNA on a plate, the length of the polynucleotide probe to be fixed on the basal plate is usually 100 to 4000 bases, preferably 200 to 4000 bases, and more preferably 500 to 4000 bases. On the other hand, to immobilize synthetic polynucleotides, the length of the probes are usually 15 to 500 bases, preferably 30 to 200 bases, and more preferably 50 to 200 bases. The step for fixing of the polynucleotides on the basal plate is also called "printing" in general. Specifically, the printing can be, for example, conducted as follows, but is not limited thereto. Several kinds of polynucleotide probes are printed within an area of 4.5 mmx 4.5 mm. According to this step, respective arrays can be printed using one pin. Accordingly, when a tool with 48 pins is used, 48 arrays can be printed repeatedly on one standard slide for microscopes.

Then, the cDNA sample is contacted with the basal plate according to the present method. The cDNA sample is hybridized with nucleotide probes on the basal plate, which can specifically hybridize with a DNA encoding a polypeptide of the present invention, in this step. Although the reaction solution and the reaction condition for hybridization varies depending on various factors, such as the length of the nucleotide probe fixed on the basal plate, they can be determined according to usual methods well known to those skilled in the art.

Next, the expression level of the gene encoding the polypeptide of the present invention contained in the cDNA sample is measured by detecting the hybridization intensity of the cDNA sample with the nucleotide probe fixed on the basal plate. Further, the measured expression level of the gene encoding the polypeptide of the present invention is compared with that of the control.

A cDNA in the cDNA sample hybridizes with the nucleotide probe fixed on the basal plate when such cDNA derived from the gene encoding the polypeptide of the present exists in the cDNA sample. Thus, the expression level of the gene encoding the polypeptide of the present invention can be measured by detecting the intensity of the hybridization of the polynucleotide probe with the cDNA. One skilled in the art can appropriately conduct the detection of the hybridization intensity of the polynucleotide probe with the cDNA depending on the kind of substances used for labeling the cDNA sample. For example, when the cDNA is labeled with a fluorescent substance, it can be detected by reading out the fluorescent signal with a scanner.

The expression level of the gene encoding the polypeptide of the present invention in cDNA samples derived from a subject and control (normal healthy subject) can be measured simulatously in one measurement by labeling them with different fluorescent substances according to the method of the present invention. For example, one of the above-mentioned cDNA samples can be labeled with a fluorescent substance, Cy5, and the other with Cy3. The intensity of respective fluorescent signals show the expression level of the gene encoding the polypeptide of the present invention in the subject and the control, respectively (Duggan et al., Nat. Genet. 21:10-14 (1999)).

On the other hand, polypeptide samples are first prepared from subjects in the test for the translational level of a gene encoding a polypeptide of the present invention. Then, the amount of the polypeptide of the present invention contained in the polypeptide sample is measured and compared with that of the control.

Exemplarily methods include the SDS polyacrylamide electrophoresis method; and methods utilizing antibodies binding to the polypeptides of the invention like the Western blotting method, dot-blotting method, immunoprecipitation method, enzyme-linked immunosorbent assay (ELISA), and immunofluorescence.

When the expression level of a gene encoding a polypeptide of the present invention is significantly changed in comparison with that of the control, the subject is judged to be infected with a disease related to the expression abnormality of the gene, or to be in danger for the onset of the disease.

### <Test drug>

Furthermore, the present invention provides test drugs for diseases related to abnormal expression of a gene encoding a polypeptide of the present invention, or related to abnormal activities of a polypeptide of the present invention.

An embodiment of a test drug of the present invention contains an oligonucleotide having a chain length of at least 15 nucleotides which hybridizes with a DNA containing a polynucleotide encoding a polypeptide of the present invention or its expression control region as mentioned above. The oligonucleotide can be used in the above-mentioned test method of the present invention as a probe for detecting the gene encoding the polypeptide of the present invention or its expression control region, or as a primer for amplifying the gene encoding the polypeptide of the present invention or its expression control region. The oligonucleotides of the present invention can be prepared, for example, by a commercially available oligonucleotide synthesizing machine. The probes can be also prepared as double-stranded DNA fragments which are obtained by restriction enzyme treatments and the like. The oligonucleotides of the present invention are preferably appropriately labeled for the use as a probe. The method of labeling includes, for example, a labeling method using T4 polynucleotide kinase to phosphorylate the 5'-terminus of the oligonucleotide with ³²P; and a method of introducing substrate bases, which are labeled with isotopes such as ³²P, fluorescent dyes, biotin, and so on using random hexamer oligonucleotides and such as primers and DNA polymerase such as Klenow enzyme (the random prime method, etc.).

Another embodiment of the test drug of the present invention is a test drug containing antibodies which binds to a polypeptide of the present invention described below. The antibodies are used to detect the polypeptide of the present invention in the above-mentioned test method of the present invention. The forms of the antibodies are not limited so long as they can detect the polypeptides of the present invention. Polyclonal antibodies and monoclonal antibodies are included as the antibodies for the test. The antibodies may be labeled according to needs.

For example, sterilized water, physiological saline, vegetable oils, surfactants, lipids, solubilizers, buffers, protein stabilizers (such as BSA and gelatin), preservatives, and such may be mixed in the above-mentioned test drugs except the effective ingredient, oligonucleotide and antibody, if necessary.

### <Antibody>

The present invention provides antibodies that bind to a polypeptide of the present invention. Herein, the term "antibodies" refers to polyclonal antibodies, monoclonal antibodies, chimeric antibodies, single-stranded antibodies, humanized antibodies, and Fab fragments including Fab or other products of the immunoglobulin expression library.

A polypeptide of the present invention or its fragment, or analogs thereof, or a cell that expresses them can be used as an immunogen for producing antibodies binding to the polypeptide of the present invention. The - antibodies are preferably immunospecific to a polypeptide of the present invention. The term "immunospecific" means that the antibody has substantially higher affinity to the polypeptide of the present invention than to other polypeptides.

The antibodies binding to a polypeptide of the present invention can be prepared by conventional methods. For example, a polyclonal antibody can be obtained as follows. A polypeptide of the present invention or a fusion protein thereof with GST is immunized to small animals such as rabbit to obtain serum. The polyclonal antibody is prepared by purifying the serum through ammonium sulfate precipitation; protein A or protein G column; DEAE ion exchange chromatography; affinity column wherein the polypeptide of the present invention are coupled; and so on. On the other hand, a monoclonal antibody, for example, can be prepared as follows. A polypeptide of the present invention is administered to small animals such as mouse and the spleen is subsequently extirpated from the mouse and ground down to separate cells. Then, the cells are fused with mouse myeloma cells using reagents such as polyethylene glycol, and clones that produce antibodies binding to the polypeptide of the present invention are selected from these fused cells (hybridoma). The obtained hybridoma is then transplanted into the peritoneal cavity of a mouse, and ascites is collected from the mouse. The monoclonal antibodies can be prepared by purifying the ascite using, for example, ammonium sulfate precipitation; protein A or protein G column; DEAE ion exchange chromatography; affinity column wherein the polypeptides of the present invention are coupled; and so on.

The antibodies of the present invention can be used for the isolation, identification, and purification of the polypeptides of the present invention and cells expressing them. The antibodies binding to a polypeptide of the present invention can be also used for determining the expression level of a polypeptide of the present invention to test for a disease related to abnormal expression of a polypeptide of the present invention.

### <Identification of Ligand, Agonist; or Antagonist>

The polypeptides of the present invention can be also used to identify ligands, agonists, or antagonists thereof. These object molecules of the identification may be naturally-occurring molecules as well as structural or functional imitated molecules, which are artificially synthesized. The polypeptides of the present invention are related to various biological functions, including many pathologies. Thus, the detection of compounds that activate the polypeptides of the present invention, and compounds that inhibit the activation of the polypeptides of the present invention is expected.

To identify ligands against the polypeptide of the present invention , a polypeptide of the present invention is first contacted with a candidate compound, and then, it is detected whether or not the candidate compound binds to the polypeptide of the present invention.

There is no limitation on the sample to be tested and such samples include, for example, various known compounds and peptides whose ligand activity to GPCRs are unknown (for example, those registered in the Chemical File) ; and random peptide groups, which were produced by utilizing the phage-display method (J. Mol. Biol. (1991) 222, 301-310). Further, culture supernatant of microorganism; natural components derived from plants and marine organisms; and so on can be used as the object of the screening. Moreover, extract from biotic tissues such as brain; extracted solutions from cells; expression products of gene libraries; and so on can be also mentioned as samples to be tested, but is not limited thereto.

According to the present method, binding of the purified polypeptides of the present invention with candidate compounds can be detected. Conventional methods, such as methods purifying compounds binding to a protein of the present invention by contacting a test sample with an affinity column of the polypeptide of the present invention; and the West-Western blotting method, can be utilized to detect binding. Candidate compounds are appropriately labeled according to these methods, and the binding with the polypeptide of the present invention-is detected utilizing the label. Further, a method detecting the surface plasmon resonance changes caused by the dissociation of a trimeric-type GTP binding protein due to the binding of a ligand, by preparing cell membranes in which the polypeptide of the present invention is expressed, fixing the membrane on a chip, and detecting the changes of surface plasmon resonance on the chip (Nature Biotechnology (99) 17:1105). Further, the binding activity of a candidate compound and the polypeptide of the present invention can be also detected using signals as an index of activation of the polypeptide of the present invention. Such signal includes, for example, changes of intracellular Ca²⁺ level, changes of intracellular cAMP level, changes of intracellular pH, and changes of intracellular adenylate cyclase level, but are not restricted to these examples.

As an example of the method, a procedure as follows can be conducted: (1) a cell membrane expressing the polypeptide of the present invention is mixed with 400 pM of GTPγS labeled with ³⁵S in a solution of 20 mM HEPES (pH 7.4), 100 mM Nacl, 10 mM MgCl₂, and 50µM GDP; (2) the reaction solution is incubated in the presence and in the absence of a test sample; (3) the solution is filtrated; and (4) the radioactivity of bound GTPγS is compared.

Further, the GPCR share a system transmitting a signal into the cell through the activation of the trimeric-type GTP binding protein in common. The trimeric-type GTP binding protein is classified depending on the type of activated intracellular transmission system into 3 types : (1) Gq type, those increasing Ca²⁺; (2) Gs type, those increasing cAMP; and (3) Gi type, those suppressing cAMP. Positive signals of the ligand screening can be transduced to an increase of the Ca²⁺ level, which is the intracellular transmission pathway of Gq, by applying the system. More specifically, it can be transduced to an increase of the Ca²⁺ level by forming chimeras of Gq protein α subunit and other G protein α subunits, or by using promiscuous G α protein, G α15 and G α16. The increased Ca²⁺ level can be detected using changes of reporter gene systems, comprising TRE (TPA responsive element) or MRE (multiple responsive element) upstream in the system; staining indicators such as Fura-2, Fluo-3; and fluorescent protein, aequorin, and so on as an index. Similarly, the chimerizing the Gs protein α subunit and other G protein α subunit to transduce the positive signals to increased CAMP levels, which is the intracellular transmission pathway of Gs, the ligands, can be detected by using the changes in a reporter gene system including CRE (CAMP-responsive element) upstream as an index (Trends Pharmacol. Sci. (99) 20:118).

Host cells to express the polypeptides of the present invention in the screening system are not specifically limited, and various host cells can be used in accordance with the object. For example, mammal cells such as COS cell, CHO cell, HEK 293 cell; yeast; Drosophila-derived cell; and *E. coli* cell be mentioned. Vectors containing a promoter positioned upstream of the gene encoding the polypeptide of the present invention, a splice site of RNA, polyadenylation site, transcription termination sequence, origin of replication, and such can be preferably used as vectors for expressing the polypeptides of the present invention in vertebrate animal cells. For example, pSV2dhfr (Mol. Cell. Biol. (1981) 1, 854-864) containing the early promoter of SV40; pEF-BOS (Nucleic Acids Res. (1990) 18, 5322); pCDM8 (Nature (1987) 329, 840-842); pCEP4 (Invitrogen); and such are useful vectors for expressing GPCR. The insertion of a DNA encoding a polypeptide of the present invention to a vector can be carried out by a ordinary method utilizing the ligase reaction with restriction enzyme sites (Current protocols in Molecular Biology, edit. Ausubel et al., (1987) Publish. John Wiley & Sons, Section 11.4-11.11). Further, the introduction of a vector to the host cell can be carried out by known methods such as the calcium phosphate precipitation method, the electroporation method (Current protocols in Molecular Biology, edit., Ausubel et al., (1987) Publish. John Wiley & Sons. Section 9.1-9.9), the Lipofectamine method (GIBCO-BRL), the FUGENE6 reagent (Boehringer Mannheim), the microinjection method, and so on.

To identify agonists of a polypeptide of the present invention, a cell expressing the polypeptide of the present invention is contacted with candidate compounds to detect whether or not the candidate compounds generate a signal, which then works as an index of activation of the polypeptide of the present invention. Namely, compounds are identified which generate a signal indicative of activation of the present polypeptide in the above-described identification method for a ligand using cells expressing the polypeptide of the present invention. Such compounds serve as agonist candidates of the polypeptide of the present invention.

To identify antagonists of a polypeptide of the present invention, a cell expressing the polypeptide of the present invention is contacted with an agonist for the polypeptide of the present invention in the presence of a candidate compound to detect whether or not the signal, which serves as an index of activation of the polypeptide of the present invention, is reduced in comparison with a case (control) where the detection is conducted in the absence of the candidate compound. Namely, compounds suppressing the generation of the signal, which serves as an index of the activation of the present polypeptide by the agonist excitation, are isolated by acting the agonist as well as the candidate compound in the above-mentioned identification method of a ligand using the cell expressing the polypeptide of the present invention. Such compounds serve as candidates of antagonist of the polypeptide of the present invention. Examples of potent antagonists of the polypeptide of the present invention includes antibodies; in some cases, polypeptides having close relation with the ligand (e.g., a ligand fragment) ; and small molecules which bind to a polypeptide of the present invention but does not induce response (therefore, the activity of the receptor is prevented).

Further, the present invention provides a kit to be used for the above-mentioned identification method. The kit includes a polypeptide of the present invention, or a cell expressing a polypeptide of the present invention, or cell membranes of the cells. The kit may include compounds serving as candidates for ligands, agonists, and antagonists of GPCR.

### <Pharmaceutical composition for treatment of disease>

The present invention provides pharmaceutical compositions for treating patients who are in need of an increase in or the suppression of the activity or expression of a polypeptide of the present invention.

An agonist of the polypeptide of the present invention, a polynucleotide of the present invention, and a vector wherein a polynucleotide of the present invention is inserted can be used as an effective ingredient of the pharmaceutical composition. for increasing the activity or expression of the polypeptide of the present invention. On the other hand, an antagonist of a polypeptide of the present invention, a polynucleotide suppressing the expression of the gene encoding the endogenous polypeptide of the present invention in vivo can be used as an effective ingredient of the pharmaceutical composition for suppressing the activity or expression of the polypeptide of the present invention. Antagonists include polypeptides of the present invention in a soluble form, which have the ability to bind to a ligand under a competitive condition with the endogenous polypeptide of the present invention. A typical example of such competitive substance is a fragment of a polypeptide of the present invention. The antisense DNAs and ribozymes mentioned above are also included as polynucleotides suppressing the expression of a gene encoding a polypeptide of the present invention.

When a therapeutic compound is used as a pharmaceutical agent, it can be administered as a pharmaceutical composition prepared by known pharmaceutical methods, in addition to directly administering the compound itself to a patient. For example, it can be formulated into a form suitable for oral or parenteral administration, such as tablet, pill, powder, granule, capsule, troche, syrup, liquid, emulsion, suspension, injection (such as liquid, and suspension), suppository, inhalant, percutaneous absorbent, eye drop, eye ointment, obtained by mixing the active ingredient with a pharmacologically acceptable support (such as excipient, binder, disintegrator, flavor, corrigent, emulsifier, diluent, solubilizer).

Administration to a patient can be typically carried out by methods known to those skilled in the art, such as infra-arterial injection, intravenous injection, subcutaneous injection, and the like. Although the dosage varies depending on the weight and age of the patient, administration methods, and the like, one skilled in the art can appropriately select an appropriate dose. Further, if the compound can be encoded by DNA, gene therapy can be also carried out through introduction of the DNA to a vector for gene therapy.

The vectors for gene therapy include, for example, viral vectors such as retroviral vectors, adenoviral vectors, adeno-associated viral vectors; and non-viral vectors such as liposomes; and so on. The objective DNA can be administered to a patient by *ex vivo* methods and *in vivo* methods utilizing such vectors.

According to the present invention, novel GPCRs, polynucleotides encoding the polypeptides, vectors containing the polynucleotides, host cells containing the vectors, and methods or producing the polypeptides have been provided. Further, methods of identifying a compound which binds to a polypeptide or modifies its activity have been provided. The polypeptides, polynucleotides, and compounds which bind to a polypeptide of the present invention or modify its activity are expected to be useful in the development of novel preventive and therapeutic drugs for diseases associated with the polypeptides of the present invention. Furthermore, according to the present invention, test methods for diseases comprising the step of detecting mutations and expression of a gene encoding a polypeptide of the present invention have been provided. GPCR is one of the molecules which is most important and remarked in the fields of the development of pharmaceutical agents and medical treatments. Novel GPCRs comprehensively provided-in the present invention are expected to make remarkable development in these fields. Thus, the present invention provides valuable information to the researchers of GPCR.

The identification of the polypeptides of the present invention is illustrated below in detail by way of Examples.

### EXAMPLE 1. Extraction of amino acid sequences from human genome data

In the first step for discovering novel GPCR genes (i.e., sequence extraction) , the present inventors selected all candidates of the 6-frame translation sequences (6F development sequence), which exist between the initiation codon and termination codon in human genome sequences. When a plurality of initiation codons (ATG) are found on the same sequence, the initiation codon giving the longest sequence was selected. On the other hand, in order to defect sequences containing plural exons, protein-coding regions (GD sequence) were discovered using the gene discovery program (GeneDecoder) (Asai, K., et al., Pacific Symposium on Biocomputing 98, pp. 228-239 (PSB98, 1998)). Since a GPCR protein contains seven transmembrane helices with a length of about 20 residues, the condition for both sequences was set to comprise 150 residues or more (> 20*7).

375,412 sequences by 6-frame translation and 95,900 sequences by the GeneDecoder were predicted from human genome draft sequences at NCBI (2001. Feb). The sequences predicted by 6-frame translation correspond to sequences without introns, and those by the GeneDecoder are mainly constituted of sequences with plural exons.

The GeneDecoder is a gene discovery program using a hidden Marcov Model (HMM), as well as information related to sequence homology and distribution of the length of exons. The program was evaluated by using Genset 98 (http://bioinformatics weizmann.ac.il/databases/gensets/Human/), which contains 462 sequences comprising plural exons, and 2,843 exons, and resulted in 97.6% sensitivity and 40.4% selectivity at the nucleotide level. On the other hand, sensitivity and selectivity for detecting a correct exon boundary was 64.2% and 21.3%, respectively.

### Example 2. Triple analysis

BLASTP (Altschul, S.F. et al., Nucleic Acids Res. 25, 3389-3402 (1997)) for searching sequences; PFAM database (Bateman, A., et al., Nucleic Acids Res. 28, 263-266 (2000)) and PROSITE databases (Bairoch, A., Nucleic Acids Res. 20, 2013-2018 (1992)) for assigning domains and motifs; and TMWindows, which is a unique algorithm written by the present inventors, and further, Mitaku method (Hirokawa, T., et al., Bioinformatics. 14, 378-379 (1998)) for predicting TMH were used in the triple analysis. Specifically, the inventors carried out the triple analysis as follows:
(1) Amino acid sequences (6F development sequences, GD sequences) obtained in the sequence extraction step were searched in SWISSPROT database using BLASTP, and sequences which coincide with known GPCR sequences with an E-value of <10⁻¹⁰ or 10⁻⁵⁰ were selected.
(2) Sequences wherein a GPCR-specific domain in PFAM database could be assigned with an E-value of <1.0 or 10⁻¹⁰ were selected from the 6F development sequences and GD sequences using HMMER program. Simultaneously, sequences wherein a GPCR-specific motif pattern in PROSITE (Bairoch, A. Nucleic Acids Res. 20, 2013-2018(1992)) database could be assigned with a P-value of <2x 10⁻³ or <10⁻⁵ were selected.
(3) The number of transmembrane helices in 6F development sequences and GD sequences was predicted using the TMWindows and Mitaku method. For example, describing the logical sum of the result obtained by TMWindows as having 7 transmembrane helices and the result obtained by the Mitaku method as having 6 to 8 transmembrane helices as {TMWindows (7) or Mitaku (6-8)}, sequences which were coincided to respective conditions prepared as {TMWindows (7) or Mitaku (6-8)}, {TMWindows (7) or Mitaku (7)}, and {TMWindows (7) and Mitaku (7)} were selected.

The programs and databases which were used in the analysis above are described in detail. PFAM is a protein domain database which was described by the hidden Marcov Model (HMM) , HMMER (Bateman, A., et al., Nucleic Acids Res. 28, 263-266 (2000)) attributes them to the sequences, and the significance is scored by the E-value. On the other hand, PROSITE is a motif pattern which is described by normal representation. The present inventors used "P-value", which was obtained by multiplying the appearance probability of respective residues, as an index in order to score the significance of attribution. For example, when the normal representation pattern is A-[T,S]-G, the P-value is P*{Pt + Ps}*P_{G}.

TMWindows is a unique program written by the present inventors and relates to TMH prediction. Herein, the hydrophobic index of Engelman-Staitz-Goldman (Engelman, D. M., et al., Annual Review of Biophysics and Biophysical Chemistry. 15, 321-353. (1986)) is allotted to every amino acid residue, and all sequences are scanned by nine different window widths (19- to 27 residues). The index was determined as the most suitable index for membrane protein analysis through the comparison of all indices contained in the AAindex database (Tomii, K. & Kanehisa, M. Protein Eng. 9, 27-36 (1996)). Continuous regions having an average hydrophobic index of >2.5 were predicted as transmembrane helices from each window width. The numbers which are predicted by each different window sets indicates a range of the numbers of the helices. On the other hand, the number of helices was predicted by the Mitaku method using physicochemical parameters.

The thresholds used in these analyses were obtained by the evaluation of respective methods by the present inventors. The reference data set used for evaluation is a sequence set obtained by excluding fragment sequences from SWISSPROT version 39 (Bairoch, A. & Apweiler, R., Nucleic Acids Res. 28, 45-48 (2000)), which contains 1,054 known GPCR sequences and 64,154 non-GPCR sequences. Specific evaluation procedures of the analytical method are shown below.
(1) 1,054 known GPCR sequences were searched in the data set for evaluation using BLASTP, and the sensitivity and selectivity related to the discrimination of accurate and inaccurate pairs were calculated for each E-value.
(2) A PFAM domain specific to GPCR was attributed to the sequences of the data set for evaluation using HMMER, and the sensitivity and selectivity of the E-values were calculated for the number of the accurate and inaccurate attribution. On the other hand, the sensitivity and selectivity of P-values were calculated for the number of the accurate and inaccurate attribution with respect to PROSITE pattern.
(3) In general, the TMH anticipation tool is not so accurate in predicting real number of helices. However, by establishing the number of helix to be predicted widely as 6 to 8, 5 to 9, or 4 to 10, and the like, the sensitivity for detecting a real seven transmembrane helix type sequence can be significantly increased. We considered four ranges: 7, 6 to 8, 5 to 9, and 4 to 10, for both TMWindows and the Mitaku method, and calculated the sensitivity and selectivity to detect areal seven transmembrane helix for all of the combinations (16 combinations) for each of them.

During the evaluation, the present inventors laid emphasis on two thresholds, namely, the best sensitivity threshold and the best selectivity threshold. The former threshold is intended to minimize the false positive to obtain a sensitivity of almost 100%. On the other hand, the latter is intended to minimize the false negative to obtain a selectivity of almost 100%.

For example, the evaluation of the threshold of BLASTP is shown in FIG. 1. The arrow on the left represents the number of pairs between GPCRs, and the arrow on the right shows the pair between GPCR and non-GPCR sequence. In the region wherein the E-value is less than 10⁻⁵⁰, almost all of the pairs were formed between GPCR sequences, excluding some unrelated pairs near the boundary region. This corresponds to the best selectivity threshold. Interestingly, these false positives were caused by the correspondence with LDL receptor domains or EGF factor domains, which are characteristic in receptors having only one transmembrane helix. When the E-value is less than 10⁻¹⁰, the number of false positives was 115, but almost all of GPCRs were within the range. The boundary region corresponds to the best sensitivity threshold.

Similarly, as summarized in Table 1, the present inventors evaluated thresholds of respective tools and generated four levels of data sets based on them.

**Table 1**

| | Level A (Best Selectivity) | Level B | Level C | Level D (Best Sensitivity) |
|---|---|---|---|---|
| BLASTP | E <10⁻⁵⁰ (99%, 100%) | E <10⁻¹⁰ (100%. 90 1%) | E <10⁻¹⁰ (100%. 90 1%) | E <10⁻¹⁰ (100%, 90 1%) |
| PFAM | E <10⁻¹⁰ (95%, 99 6%) | E <1.0 (100%, 84 3%) | E <1.0 (100%, 84 3%) | E <1.0 (100%, 84.3%) |
| PROSITE | P <10⁻⁵ (90%, 100%) | P <2 × 10⁻³ (100%, 95 0%) | P <2 × 10⁻³ (100%, 95 0%) | P <2 × 10⁻³ (100%, 95 0%) |
| TMH Prediction | Not used | {TMWindows(7) and Mitaku(7)} (360%. 70 6%) | {TMWindows(7) or Mitaku(7)} (86.8%, 44 6%) | {TMWindows(7) or Mitaku(6-8)} (99.3%, 28 8%) |

Herein, the sensitivity (left) and selectivity (right) obtained by using each threshold are represented in the parentheses under the threshold of each program.

The most reliable data (level A, the best selectivity data set) was obtained by the logical sum of sequences obtained from the best selectivity thresholds of BLASTP, PFAM, and PROSITE. In addition, in order to discover far-related GPCR sequences, the logial sum of results by three levels (Table 1) of TMH prediction threshold and results by the best sensitivity thresholds of BLASTP, PFAM, and PROSITE was obtained. Then, the most sensitive data set was prepared as the best sensitivity data set (level D) . According to the evaluation method used by the present inventors, any of the sequences discovered by the best selectivity data set is a protein having seven transmembrane helices, and the possibility that they are a guanosine triphosphate binding protein-coupling type is extremely high.

### Example 3. Accurate selection of the number of genes

GPCR candidate substances were screened from sequences generated in the first step, using the thresholds shown in Table 1. However, since these sequences contained following duplicated examples, it was required to finally select rigidly the number of candidates.

### Case 1: Perfect matching or duplication at a same gene locus.

These resulted from using two sequence preparation methods: namely, (1) 6-frame translation, and (2) prediction by the GeneDecoder. The present inventors regarded them as same genes.

### Case 2: Many copies on different chromosomes or at different positions on a same chromosome.

From a biological viewpoint, the present inventors regarded them as different genes. Duplicated genes were most frequently found between chromosome 2 and 11.

### Case 3: Two or more sequences partially corresponding to any long known sequence.

These were considered to be generated by missplicing by the gene discovery program. The present inventors considered that they should be fused as generally one gene.

The present inventors first improved the precision of candidate genes by studying above-mentioned cases, respectively. Two sequences, i and j, were regarded as the same gene by using a specific algorithm: Cᵢ = Cⱼ, Fᵢ = Fⱼ, n₁ = nⱼ, and eᵢ - tj < 0 (i < j ) ; wherein 50 or more residues are aligned at 99% or more similarity (herein, "C" represents chromosome number; "F" frame number; "R" the position on a genomic sequence; and S (C,F,R) sequence), (Herein, when n is a contiguous number and t and e are relative positions at the N- and C-terminus on a contiguous sequences, the positions R is R (n, t, e)) .

After the above screening, the present inventors finally obtained the best selectivity and the best sensitivity data sets containing 827 and 2109 sequences, respectively, and also obtained other levels of data sets by considering biological information, using NCBI human draft sequences (both 2001 and 2002 version) . The number of GPCR candidates of every chromosome is summarized in Table 2 for each data set.

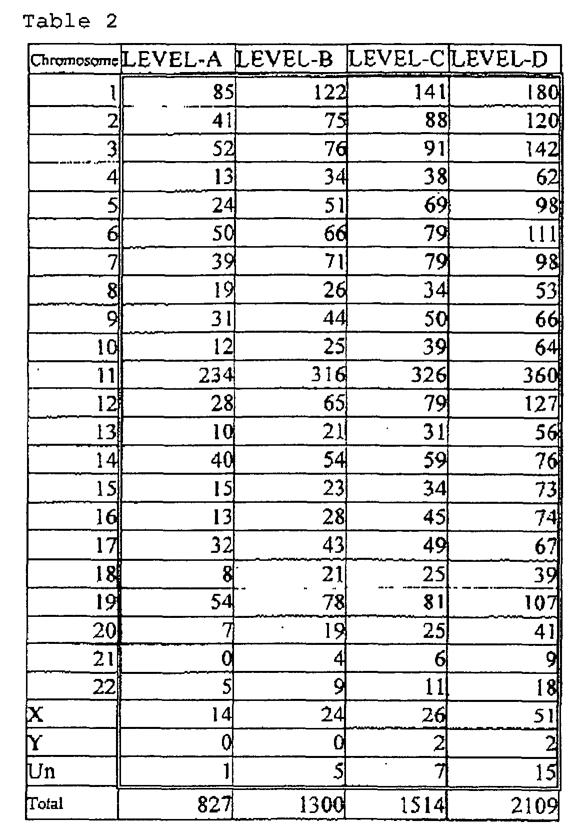

The number of GPCR candidates of every chromosome Un means those whose chromosome number is unknown.

As shown in the table, it was found that chromosome 11 has the maximum number of GPCR candidates in all levels of data sets, and chromosomes 1, 6 and, 19 also have many GPCR candidates. On the other hand, chromosomes 21 and Y have extremely few GPCR candidates. Further, this tendency does not have changed, even after updating the data monthly.

Further analysis concerning the best selectivity data set is summarized in Table 3.

**Table 3**

| Families | Data |
|---|---|
| | Total |
| Acetylchline (muscarinic) receptors | 9 |
| Adenosine and adenine nucleotide receptors | 18 |
| Adrenergic, Dopamine, Serotonine receptors | 37 - |
| Angiotensin receptors | 5 |
| Bradykinin receptors | 3 |
| Cannabinoids receptors | 1 |
| Chernokines and chemotactïc factors receptors | 31 |
| Cholecystokinin/gastrin receptors | 2 |
| Endothelin receptors | 2 |
| Family 2(B) receptors | 18 |
| Family 3(C ) receptors | 28 |
| Family fz/srno receptors | 10 |
| Glycoprotein hormone receptors | 5 |
| Histamine receptors | 2 |
| Melanocortins receptors | 5 |
| Mclanotonin receptors | 3 |
| Neuropeptide Y receptors | 6 |
| Neurotensin receptors | 4 |
| No swissprot 7TM | 17 |
| Odorant/olfactory and gustatory receptors | 507 |
| Opioid peptides receptors | 5 |
| Opsins | 5 |
| Orphan receptors | 68 |
| Other receptors | 4 |
| Platelet activating factor receptors | 3 |
| Prostanoids receptors | 8 |
| Proteinase-activated receptors | 5 |
| Releasing hormones receptors | 3 |
| Somatostatin receptors | 6 |
| Tachykinin receptors | 3 |
| Vasopressin/oxytocin receptors | 4 |
| Total | 827 |

The present inventors classified sequences by a sequence similarity of 30%, which is generally considered to be the threshold for an evolutionarily related family. The largest family is the olfactory receptor family, containing 507 members. Major families containing more than 20 members are: the adrenalin, dopamine, and serotonin receptor family (37); the 2B receptor family (18); the 3C receptor family (28) ; the chemokine and chemoatractant receptor family (31); and the orphan receptor family (68).

### Example 4. Extraction of novel sequence

Sequences were searched in UNIGENE (Schuler, G. D., J. Mol. Med. 75, 694-698 (1997)) and nr-aa (ftp://ncbi.nlm.nih.gov/blast/db/README) databases. When at least 100 or more residues in the sequences which were investigated were continuously aligned with known sequences, and when the amino acid identity of that region is 96% or more, the present inventors designated the sequence as a known sequence. Novel GPCR candidates were obtained using this standard. These data sets will be maintained and updated by routine recalculations to the future.

The present inventors classified the extracted novel sequences into groups A, B, and C (Table 4 to Table 6) - The sequences in groups A, B, and C are newly identified sequences, selected based on the search method in UNIGENE and nr-aa database, after the numbers of the sequences were made precise based on the best selectivity data set (level A), the data set at level B, and the data set at level C, respectively, among sequence sets which were obtained by triple analysis.

Further, the nucleotide sequences and amino acid sequences of the novel gene described in group A are shown in SEQ ID NOs: 1 to 936; those described in group B are shown in SEQ ID NOs: 1 to 1684; and those described in C group are shown in SEQ ID NOs: 1 to 2070.

**Table 4**

| | Assayed amino acids | Assay method | Number of novel genes | SEQ ID NO; |
|---|---|---|---|---|
| A-1 | 6F development sequence | Homology search | 241 | 1 ― 482 |
| A-2 | GD sequence | Homology search | 113 | 483 - 708 |
| A-3 | 6F development sequence + GD sequence | Motif search Domain search | 114 | 709 ― 936 |
| A-1 Sequence set obtained through the assay of 6F sequences by homology search (use of the most easy method). | | | | |
| A-2 The part of amino acid sequence comprising multi exon, increased by use of GD sequence. | | | | |
| A-3 Sequence set found for the first time by use of motif and domain attribution. Homologous with very little homology, which cannot be found through normal sequence searches, were detected. | | | | |

**Table 5**

| | Assayed amino acids | Assay method | Number of novel genes | SEQ ID NO: |
|---|---|---|---|---|
| B-1 | 6F development sequence | Homology search | 378 | 1 - 482 937 ― 1210 |
| B-2 | GD sequence | Homology search | 180 | 483 ― 708 1211 ― 708 |
| B-3 | 6F development sequence + GD sequence | Motif search Domain search | 259 | 709 ― 936 1345 - 1634 |
| B-4 | 6F development sequence + GD sequence | Transmembrane helix prediction | 25 | 1635 - 1684 |
| B-1 Sequence set obtained through the assay of 6F sequences by homology search (use of the most easy method) | | | | |
| B-2 The part of amino acid sequence comprising multi exon, increased by use of GD sequence. | | | | |
| B-3 Sequence set found for the first time by use of motif and domain attribution. Homologous with very little homology, which cannot be found through normal sequence searches, were detected. | | | | |
| B-4 Sequence set found for the first time by use of the prediction method for the transmembrane helix. Sequences which cannot be found even through normal homology search, motif and'domain attribution were also determined. | | | | |

**Table 6**

| | Assayed amino acids | Assay method | Number of novel genes | SEQ ID NO; |
|---|---|---|---|---|
| C-1 | 6F development sequence | Homology search | 378 | 1 - 482 937 - 1210 |
| C-2 | GD sequence | Homology search | 180 | 483 - 708 1211 - 708 |
| C-3 | 6F development sequence + GD sequence | Motif search Domain search | 259 | 709 - 936 1345 - 1634 |
| C-4 | 6F development sequence + GD sequence | Transmembrane helix prediction | 218 | 1635 - 2070 |
| C-1 Sequence set obtained through the assay of 6F sequences by homology search (use of the most easy method). | | | | |
| C-2 The part of amino acid sequence comprising multi exon, increased by use of GD sequence. | | | | |
| C-3 Sequence set found for the first time by use of motif and domain attribution. Homologous with very little homology, which cannot be found through normal sequence searches, were detected. C-4 Sequence set found for the first time by use of the prediction method for the transmembrane helix. Sequences which cannot be found even through normal homology search, motif and domain attribution were also determined. | | | | |

## Claims

1. A polynucleotide encoding a guanosine triphosphate-binding protein coupled receptor selected from the group of:
(a) a polynucleotide encoding a polypeptide comprising an amino acid sequence selected from the group consisting of the even-numbered SEQ ID NOs from SEQ ID NO: 2 to SEQ ID NO: 2070;
(b) a polynucleotide comprising a coding region of the nucleotide sequence selected from the group consisting of the odd-numbered SEQ ID NOs from SEQ ID NO: 1 to SEQ ID NO: 2069;
(c) a polynucleotide encoding a polypeptide comprising an amino acid sequence selected from the group consisting of the even-numbered SEQ ID NOs from SEQ ID NO: 2 to SEQ ID NO: 2070 wherein one or more amino acid residues are substituted, deleted, added and/or inserted; and
(d) a polynucleotide hybridizing under stringent conditions with a DNA consisting of a nucleotide sequence selected from the group consisting of the odd-numbered SEQ ID NOs from SEQ ID NO: 1 to SEQ ID NO: 2069.

2. A polynucleotide encoding a fragment of a polypeptide comprising an amino acid sequence selected from the group consisting of the even-numbered SEQ ID NOs from SEQ ID NO: 2 to SEQ ID NO: 2070.

3. A vector comprising the polynucleotide of claim 1 or 2.

4. A host cell retaining the polynucleotide of claim 1 or 2, or the vector of claim 3.

5. A polypeptide encoded by the polynucleotide of claims 1 or 2.

6. A method for producing the polypeptide of claim 5, comprising the step of culturing the host cell of claim 4, and recovering the produced polypeptide from said host cell or culture supernatant thereof.

7. An antibody binding to the polypeptide of claim 5.

8. A method of identifying a ligand of the polypeptide of claim 5, comprising the steps of:
(a) contacting a candidate compound with the polypeptide of claim 5, a cell expressing the polypeptide of claim 5, or a cytoplasmic membrane of the cell; and
(b) detecting whether the candidate compound binds to the polypeptide of claim 5, the cell expressing the polypeptide of claim 5, or the cytoplasmic membrane thereof, wherein the detection of binding implies that said candidate compound is a ligand of the polypeptide of claim 5.

9. A method for identifying an agonist of the polypeptide of claim 5, comprising the steps of:
(a) contacting a candidate compound with a cell expressing the polypeptide of claim 5; and
(b) detecting whether the candidate compound induces a signal that indicates the activation of the polypeptide of claim 5, wherein the detection of activation implies that said candidate compound is an agonist of the polypeptide of claim 5.

10. A method for identifying an antagonist of the polypeptide of claim 5, comprising the steps of:
(a) contacting a cell expressing the polypeptide of claim 5 with an agonist of the polypeptide of claim 5 in the presence of a candidate compound; and
(b) detecting whether the intensity of the signal that indicates the activation of the polypeptide of claim 5 is reduced or not by comparing with the signal detected in the absence of the candidate compound, wherein the detection of a reduction in intensity implies that said candidate compound is an antagonist of the polypeptide of claim 5..

11. A ligand identified by the method of claim 8.

12. An agonist identified by the method of claim 9.

13. An antagonist identified by the method of claim 10.

14. A kit used for the method of any one of claims 8 to 10, comprising at least one molecule selected from the group:
(a) the polypeptide of claim 5; and
(b) the host cell of claim 4 or cytoplasmic membrane thereof.

15. A pharmaceutical composition for treating a patient, who is in need of increased activity or expression of the polypeptide of claim 5, comprising an effective amount of a molecule for the treatment selected from the group of:
(a) an agonist of the polypeptide of claim 5;
(b) the polynucleotide of claims 1 or 2; and
(c) the vector of claim 3.

16. A pharmaceutical composition for treating a patient having an endogenous activity or expression of the polypeptide of claim 5 that needs to be suppressed, comprising an effective amount of a molecule for the treatment selected from the group of:
(a) an antagonist of the polypeptide of claim 5; and
(b) a polynucleotide suppressing the expression of a gene encoding the endogenous polypeptide of claim 5 *in vivo.*

17. A method for testing a disorder associated with the aberration in the expression of a gene encoding the polypeptide of claim 5 or the aberration in the activity of the polypeptide of claim 5 in a subject, comprising the step of detecting a mutation in the gene or in the expression control region thereof of the subject.

18. The method for testing of claim 17, comprising the steps of:
(a) preparing a DNA sample from a subject;
(b) isolating the DNA encoding the polypeptide of claim 5 or the expression control region thereof;
(c) determining the nucleotide sequence of the isolated DNA; and
(d) comparing the nucleotide sequence of DNA determined in step (c) with that determined in a control.

19. The method for testing of claim 17, comprising the steps of:
(a) preparing a DNA sample from a subject;
(b) cleaving the prepared DNA sample with a restriction enzyme;
(c) separating DNA fragments according to the sizes thereof; and
(d) comparing the detected sizes of the DNA fragments with those detected in a control.

20. The method for testing of claim 17, comprising the steps of:
(a) preparing a DNA sample from a subject;
(b) amplifying the DNA encoding the polypeptide of claim 5 or the expression control region thereof from the DNA sample;
(c) cleaving the amplified DNAs with a restriction enzyme;
(d) separating the DNA fragments according to the sizes thereof; and
(e) comparing the detected sizes of the DNA fragments with those detected in a control.

21. The method for testing of claim 17, comprising the steps of:
(a) preparing a DNA sample from a subject;
(b) amplifying the DNA encoding the polypeptide of claim 5 or the expression control region thereof from the sample;
(c) dissociating the amplified DNA to single-stranded DNAs;
(d) separating the dissociated single-stranded DNAs on a non-denaturing gel; and
(e) comparing the mobility of the separated single-stranded DNAs with that of a control.

22. The method for testing of claim 17, comprising the steps of:
(a) preparing a DNA sample from a subject;
(b) amplifying the DNA encoding the polypeptide of claim 5 or the expression control region thereof from the sample;
(c) separating the amplified DNAs on a gel with increasing concentration gradient of a DNA denaturant; and
(d) comparing the mobilities of the separated DNAs with those of a control.

23. A method for testing disorders associated with the aberration in the expression of a gene encoding the polypeptide of claim 5, comprising the step of detecting the expression level of the gene in the subject.

24. The method for testing of claim 23, comprising the steps of:
(a) preparing an RNA sample from a subject;
(b) measuring the amount of RNA encoding the polypeptide of claim 5 contained in said RNA sample; and
(c) comparing the amount of measured RNA with that measured in a control.

25. The method for testing of claim 23, comprising the steps of:
(a) providing a cDNA sample prepared from a subject and a basal plate on which nucleotide probes hybridizing to the DNA encoding the polypeptide of claim 5 are immobilized;
(b) contacting said cDNA sample with said basal plate;
(c) measuring the expressed amount of the gene encoding the polypeptide of claim 5 contained in said cDNA sample by detecting the hybridization intensity between said cDNA sample and the nucleotide probe immobilized on the basal plate; and
(d) comparing the measured expression amount of the gene encoding the polypeptide of claim 5 with the expression measured for a control.

26. The method for testing of claim 23, comprising the steps of:
(a) preparing a protein sample from a subject;
(b) measuring the amount of the polypeptide of claim 5 contained in said protein sample; and
(c) comparing the amount of the measured polypeptide with that measured for a control.

27. An oligonucleotide having a chain length of at least 15 nucleotides hybridizing to a DNA encoding the polypeptide of claim 5 or the expression control region thereof.

28. An assay reagent for testing disorders associated with aberration in the expression of the gene encoding the polypeptide of claim 5 or aberration in the activity of the polypeptide of claim 5, comprising the oligonucleotide of claim 27.

29. An assay reagent for testing disorders associated with aberration in the expression of a gene encoding the polypeptide of claim 5 or aberration in the activity of the polypeptide of claim 5, comprising the antibody of claim 7.
